(19) Europäisches Patentamt
European Patent Office
Office européen des brevets

(11) EP 3 211 083 A1

(12) EUROPEAN PATENT APPLICATION

(43) Date of publication:
30.08.2017 Bulletin 2017/35

(51) Int Cl.:
*C12N 15/115* (2010.01)

(21) Application number: 16305224.4

(22) Date of filing: 26.02.2016

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: Centre National de la Recherche Scientifique
75016 Paris (FR)

(72) Inventors:
- RYCKELYNCK, Michael
67100 STRASBOURG (FR)
- AUTOUR, Alexis
67000 STRASBOURG (FR)
- WESTHOF, Eric
67000 STRASBOURG (FR)

(74) Representative: Monni, Richard
Cabinet LLR
11 boulevard de Sébastopol
75001 Paris (FR)

# (54) FLUORESCENT APTAMERS AND THEIR APPLICATIONS

(57) The present invention relates to a nucleic acid molecule capable of binding to a fluorophore molecule, the nucleic acid molecule being a molecule containing a first and a second region, said first and second regions being such that :
- the first region comprises the nucleotide sequence of SEQ ID NO: 1; and
- the second region comprises the nucleotide sequence of SEQ ID NO: 2.

EP 3 211 083 A1

## Description

The present invention concerns new aptamers and their applications.

The recent development of new fluorogenic dyes and the concomitant isolation of specific RNA aptamers (1-10) offer the possibility to monitor in real-time the synthesis of a specific RNA both in vitro and in vivo with applications as diverse as live-cell imaging (11-13), biosensing (14,15) or screening (16). Among this new generation of dyes, the 3,5-difluoro-4-hydroxybenzylidene imidazolinone (DFHBI), a commercially available dye mimicking the natural fluorophore of the green fluorescent protein (17), proved to be particularly well suited for live-cell imaging as it is non toxic, cell membrane-permeable, does not interact with cell components and has a low fluorescence in it is free state (18). These attractive properties led to the isolation of a first DFHBI-binding aptamer termed Spinach (6). While Spinach was able to enhance DFHBI fluorescence more than ~2000 times upon binding, it however suffered from several limitations such as a limited folding efficiency and thermal instability. These limitations were partly overcome in a second version of the aptamer (Spinach2) obtained by rational design (19).

Structural studies shed light on the recognition mechanism between the dye and both RNA (20,21) by revealing that both aptamers were built around a G-quadruplex structure and that DFHBI was recognized in a pocket encompassing the top G-quartet, a base triple and a unpaired G residue. The stabilization of this structure requires potassium ions, making the fluorescence of the complex sensitive to the nature of the cation present in the reaction mixture (20,21).

Like every artificial RNA aptamer discovered to date, Spinach was initially isolated by SELEX, a powerful approach to identify specific and high affinity RNA ligand starting from large libraries (22-24) but that does not allow to select for fluorescence phenotypes. Consequently, light-up aptamers are usually identified across a second labor-intensive and low throughput post-selection screening step that allows exploring only a limited fraction of the selected variants. Therefore, and as confirmed by a recent study (25), Spinach and its derivatives were likely to be sub-optimal fluorogenic aptamers. This was further supported by a recent work in which FACS-based screening of SELEX-enriched libraries led to the isolation of Broccoli, a DFHBI-binding aptamer optimized for in vivo applications with superior stability and fluorescence properties (4).

Beside live-cell imaging applications, several new in vitro fluorogenic assays were developed using either the full-length (16,26) or a split version of DFHBI-binding RNA aptamers (27) and Spinach was even used as extracellular metabolite fluorogenic sensor (28). However, despite its great potential, little was known about how well Spinach and its derivatives were adapted to in vitro conditions.

The present invention is directed to overcoming these and other deficiencies in the art.

One aim of the invention is to provide new aptamers that have enhanced properties compared to the previously proposes ones.

Another aim of the invention is to provide use of these aptamers.

The present invention relates to a nucleic acid molecule capable of binding to a fluorophore molecule, the nucleic acid molecule being a molecule containing a first and a second region, said first and second regions being such that:

- the first region comprises the nucleotide sequence of SEQ ID NO: 1 : UGAGGGUCGGGUCC; and
- the second region comprises the nucleotide sequence of SEQ ID NO: 2 : GUUGAGUAGAGUGUGGGCU,

preferably wherein the first and the second regions present at least a partial base pairing.

In other words, the invention relates to a nucleic acid molecule capable of binding to a fluorophore molecule, the nucleic acid molecule being a double strand nucleic acid molecule in which

- the first strand comprises the nucleotide sequence of SEQ ID NO: 1, and
- the second strand comprises the nucleotide sequence of SEQ ID NO: 2,

preferably, wherein the first and the second strand present at least a partial base pairing.

In another definition , the invention relates to a nucleic acid molecule capable of binding to a fluorophore molecule, the nucleic acid molecule being in a conformation wherein it contains two strands in juxtaposition, wherein

- the first strand comprises the nucleotide sequence of SEQ ID NO: 1, and
- the second strand comprises the nucleotide sequence of SEQ ID NO: 2, preferably,

wherein the first and the second strand present at least a partial base pairing.

The invention is based on the unexpected observation made by the inventors that specific aptamers having some mutations compared to the previously discloses Spinach sequence harbor enhanced properties.

The present invention also relates to nucleic acid molecules that are known in the art as aptamers. Aptamers are nucleic acid molecules characterized by a secondary structure that may possess one or more stems {i.e., base-paired regions) as well as one or more non base-paired regions along the length of the stem. These non base-paired regions can be in the form of a bulge or loop {e.g., internal loop) along the length of the stem(s) and/or a loop at the end of the one or more stem(s) {e.g., hairpin loop). These nucleic acid aptamers possess specificity in binding to a particular target molecule, and they noncovalently bind their target molecule through an interaction such as an ion-ion force, dipole-dipole force, hydrogen bond, van der Waals force, electrostatic interaction, stacking interaction or any combination of these interactions.

The inventors found that a mutation within the central region of Spinach enhances significantly the properties of the aptamer and the resulting fluorescence when interacting with a fluorophore.

The first and the second regions of SEQ ID NO: 1 and SEQ ID NO: 2 constitute the minimal essential domain of the aptamer responsible of the fluorescence properties.

Advantageously, the invention relates to the nucleic acid molecule as defined above, said nucleic acid molecule being a linear single-stranded molecule, a circular single-stranded molecule or a two-stranded molecule.

As discloses in the art, the aptamer according to the invention may be a linear single-stranded molecule. The sequences SEQ ID NO: 1 and SEQ ID NO: 2 are separated from each other in the same molecule but are close to each other when the molecule acquires its final tridimensional conformation.

Moreover, the aptamer can be a circular single-stranded molecule. In this case, the molecule has the same structure than a linear single-stranded molecule, except that the 5'- and 3'- ends are linked by a phosphodiester bond.

The aptamer according to the invention can also be constitutes by two separated molecules, the first one containing the sequence SEQ ID NO: 1 and the second one containing the sequence SEQ DI NO: 2, these two molecules being close to each other to confers the molecule a structure similar to the structure adopted by a single stranded molecule.

In the invention, when the aptamer is a single stranded linear or circular molecule, both sequences are contained in the same molecule. By contrast, when the aptamer is constituted by two different single stranded molecules, each sequences is contained in one specific molecule, i.e. the two sequences cannot be contained by the same single stranded molecule.

Advantageously, the invention relates to the nucleic acid molecule as defined above, wherein the nucleic acid molecule comprises one of the nucleotide sequences of

- $(N)_a$UGAGGGUCGGGUCC$(N)_b(N)_c(N)_b$GUUGAGUAGAGUGUGGGCU$(N)_a$ (SEQ ID NO: 3),
- $(N)_a$GUUGAGUAGAGUGUGGGCU$(N)_b(N)_c(N)_b$UGAGGGUCGGGUCC$(N)_a$ (SEQ ID NO: 4), or

the two following sequences

- $(N)_a$UGAGGGUCGGGUCC$(N)_b(N)_d$ (SEQ ID NO: 5),
- $(N)_a$GUUGAGUAGAGUGUGGGCU $(N)_b(N)_d$ (SEQ ID NO: 6),

wherein a, b , c and d are integer

a is higher than or equal to 4, preferably varies from equals to 4 to 100,
b is higher than or equal to 5, preferably varies from 5 to 50,
c is higher than or equal to 4, preferably varies from 4 to 200,

and 2d=c.

As it is well known in the art, N designates any nucleotides, i.e. either A, or U (for RNA) or T (For DNA), or G or C.

In the above sequences, a varies from 7 to 100, which means that a can be equal to 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100.

In the above sequences, c varies from 5 to 50, which means that b can be equal to 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 and 50.

In the above sequences, c varies from 4 to 200, which means that c can be equal to 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199 and 200.

In the above sequences, d equals half a c. This concerns only the aptamers that are constituted by two different single stranded molecules.

In one advantageous embodiment, the invention relates to the nucleic acid molecule defined above, wherein the nucleic acid molecule comprises one of the nucleotide sequence of

- SEQ ID NO: 7 :

  NNNNNNNNNNUGAGGGUCGGGUCCNNNNNNnnnnNNNNNNGUUGAGUAGAGUGUGGGCUNNNNNNNNNN, or

- SEQ ID NO: 8:

  NNNNNNNNGUUGAGUAGAGUGUGGGCUNNNNNNNNnnnnNNNNNNNNNNUGAGGGUCGGGUCCNNNNNNNN, or

- both sequences of SEQ ID NO: 9 (NNNNNNNNNNUGAGGGUCGGGUCCNNNNNNnn) and SEQ ID NO: 10 (NNNNNNNNNNGUUGAGUAGAGUGUGGGCUNNNNNNnn).

More advantageously, the invention relates to the nucleic acid molecule as defined above, wherein the nucleic acid molecule consists or consists essentially of the nucleotide sequence of SEQ ID NO: 11, i.e. the sequence of : 5'-GCGACUACGGUGAGGGUCGGGUCCAGUAGCUUCGGCUACUGUUGAGUAGAGU GUGGGCUCCGUAGUCGC -3'. This molecule is called iSpinach.

In one another advantageous embodiment, the invention relates to the nucleic acid molecule above-defined, wherein the fluorophore molecule is a fluorophore comprising a methyne bridge between a substituted aromatic ring system and a substituted imidazol(thi)one, oxazol(thi)one, pyrrolin(thi)one, or furan(thi)one.

Subclasses of these fluorophores, including oxazolithiones, pyrrolinthiones, imidazolithiones, and furanthiones, as well as those possessing an oxazolone ring, imidazolone ring, furanone ring, or pyrrolinone ring, are shown and/or described in the applications WO 2010/096584 and WO 2013/016694.

Without limitation the following fluorophores, 4-(3,4,5-trimethoxybenzylidene)-1,2-dimethyl-imidazol-5-one ("TMBI"); 4-(4-hydroxy-3,5-dimethoxybenzylidene)-1,2-dimethyl-imidazol-5-one ("DMHBI"); 4-(3,5-difluoro-4-hydroxybenzylidene)-1,2-dimethyl-imidazol-5-one ("DFHBI"); (5Z)-5-[(3,5-Difluoro-4-hydroxyphenyl)methylene]-3,5-dihydro-2-methyl-3-(2,2,2-trifluoroethyl)-4*H*-imidazol-4-one ("DFHBI-1T") (E)- 4-(3,5-difluoro-4-hydroxybenzylidene)-l-methyl-5-oxo-4,5-dihydro-IH-imidazole-2-carbaldehyde O-methyl oxime ("DFHBI-methyloxime"); 4-(3,5-dichloro-4-hydroxybenzylidene)-1,2-dimethyl-imidazol-5-one; 4-(3,5-dibromo-4-hydroxybenzylidene)- 1 ,2-dimethyl-imidazol-5-one; 4-(2-hydroxybenzylidene)- 1,2-dimethyl-imidazol-5-one ("o-HBI"); 4-(2-methoxybenzylidene)- 1 ,2-dimethyl- imidazol-5-one; 4-(3-fluoro-4-hydroxy-5-methoxybenzylidene)- 1 ,2-dimethyl- imidazol-5-one; 4-(4-(dimethylamino)benzylidene)-1,2-dimethyl-imidazol-5-one ("DMABI"); 4-(4-(t-butylthio)benzylidene)-l ,2-dimethyl-imidazol-5-one; 4-(4- (methylthio)benzylidene)- 1,2-dimethyl-imidazol-5 -one; 4-(4-cyanobenzylidene)- 1 ,2- dimethyl-imidazol-5-one; 4-(3,5-difluoro-4-acetate)benzylidene-1,2-dimethyl- imidazol-5-one; 4-(4-hydroxy-3-nitrobenzylidene)- 1 ,2-dimethyl-imidazol-5-one; 4- (4-hydroxy-3-methoxy-5-nitrobenzylidene)-1,2-dimethyl-imidazol-5-one; 4-(4- methoxy-3-nitrobenzylidene)- 1,2-dimethyl-imidazol-5-one; 4-(4-bromobenzylidene)- 1 ,2-dimethyl-imidazol-5-one; 4-(4-chlorobenzylidene)-l ,2-dimethyl-imidazol-5-one; 4-(4-hydroxybenzylidene)- 1,2-dimethyl-imidazol-5-one ("p-HBI"); 4-((indol-7-yl)methylene)-1,2-dimethyl-imidazole-5-one; 4-((indol-3-yl)methylene)-1,2- dimethyl-imidazole-5-one; 4-((indol-3-yl)methylene)-1-methyl-2-phenyl-imidazole-5- one; 4-(4-hydroxy-3,5-dimethoxybenzylidene)-l-methyl-2-phenyl-imidazole-5-one; 4-(4-(dimethylamino)benzylidene)- 1 -methyl -2-phenyl-imidazole-5 -one; 4-(4-hydroxybenzylidene)-2-acetyl-1-methyl-imidazole-5 -one; 4-(4-hydroxybenzylidene)- 1-methyl-2-prop-l-enyl-imidazole-5-one; 3-(4-(4-hydroxybenzylidene)-4,5-dihydro- l-methyl-5-oxo-imidazol-2-yl)acrylamide; 3-(4-(4-hydroxybenzylidene)-4,5-dihydro- l-methyl-5-oxo-imidazol-2-yl)acrylic acid; and methyl 3-(4-(4-hydroxybenzylidene)- 4,5-dihydro-l-methyl-5-oxo-imidazol-2-yl)acrylate can be used in the invention. Of these, DFHBI and DFHBI- methyloxime are particularly desirable because of their distinct emission maxima and high quantum yield.

More advantageously, the above fluorophore molecule is 4-(3,5-difluoro-4-hydroxybenzylidene)-1,2-dimethyl-imidazol-5-one ("DFHBI").

The invention also relates to a molecular complex comprising, consisting essentially of or consisting of :

- a fluorophore molecule comprising a methyne bridge between a substituted aromatic ring system and a substituted imidazol(thi)one, oxazol(thi)one, pyrrolin(thi)one, or furan(thi)one, in particular DFHBI ; and
- the nucleotide molecule as defined above specifically bound to the fluorophore molecule.

In an advantageous embodiment, the invention relates to molecular complex comprising, consisting essentially of or consisting of:

- a fluorophore molecule which is DFHBI ; and
- the nucleotide molecule comprising or consisting essentially of the sequences of the group consisting of SEQ ID NO : 1, SEQ ID NO : 2, SEQ ID NO: 3, SEQ ID NO : 4, SEQ ID NO : 5, SEQ ID NO : 6, SEQ ID NO : 7, SEQ ID NO: 8, SEQ ID NO: 9, SEQ ID NO: 10 and SEQ ID NO: 11.

The above complex exhibits remarkable resistance to photobleaching.

More advantageously, the invention relates to the complex as defined above, wherein the fluorophore molecule has substantially enhanced fluorescence, in comparison to the fluorophore molecule prior to specific binding, upon exposure to radiation of suitable wavelength.

Within the context of DFHBI, the suitable activation wavelength is from about 460 nm to 470 nm, in particular from 465 nm to 469 nm. When interaction with the aptamer according to the invention is carried out, the maximal emission is about 501 nm.

The invention further relates to deoxyribonucleotide molecule, or DNA molecule, coding for a nucleic acid molecule as defined above.

The sequences of the DNA molecules encompasses by the invention are :

SEQ ID NO : 12 TGAGGGTCGGGTCC codes for SEQ ID NO : 1,
SEQ ID NO : 13 GTTGAGTAGAGTGTGGGCT codes for SEQ ID NO : 2,
SEQ ID NO : 14 NTGAGGGTCGGGTCCNNNGTTGAGTAGAGTGTGGGCTN codes for SEQ ID NO : 3,
SEQ ID NO : 15 NGTTGAGTAGAGTGTGGGCTNNNTGAGGGTCGGGTCCN codes for SEQ ID NO : 4,
SEQ ID NO : 16 NTGAGGGTCGGGTCCNN codes for SEQ ID NO : 5,
SEQ ID NO : 17 NGTTGAGTAGAGTGTGGGCTNN codes for SEQ ID NO : 6,
SEQ ID NO: 18 NNNNNNNNNNTGAGGGTCGGGTCCNNNNNNNNNNNNNNNNGTTGAGTAGAGTGTGGGCTNNNNNNNNNN codes for SEQ ID NO : 7,
SEQ ID NO : 19 NNNNNNNNGTTGAGTAGAGTGTGGGCTNNNNNNNNNNNNNNNNNNNNNNTGAGG GTCGGGTCCNNNNNNNN codes for SEQ ID NO : 8,
SEQ ID NO : 20 NNNNNNNNNNTGAGGGTCGGGTCCNNNNNNNN codes for SEQ ID NO: 9,
SEQ ID NO : 21 NNNNNNNNNNGTTGAGTAGAGTGTGGGCTNNNNNNNN codes for SEQ ID NO : 10, and
SEQ ID NO : 22 GCGACTACGGTGAGGGTCGGGTCCAGTAGCTTCGGCTACTGTTGAGTAGAGTGTG GGCTCCGTAGTCGC codes for SEQ ID NO : 11.

Advantageously, the invention relates to the DNA molecule as defined above, wherein the nucleic acid molecule is operably linked to at least one element allowing its expression, i.e. the transcription.

Transcription of the DNA molecule of the present invention is often dependent upon the presence of a promoter, which is a DNA sequence that directs the binding of RNA polymerase and thereby promotes RNA synthesis. Accordingly, the DNA molecule of the present invention may include a promoter operably coupled to the first region to control expression of the RNA aptamer. Because not all polymerases require promoters, the promoter sequence is optional.

The DNA sequences of eukaryotic promoters differ from those of prokaryotic promoters.

Furthermore, eukaryotic promoters and accompanying genetic signals may not be recognized in or may not function in a prokaryotic system and, further, prokaryotic promoters are not recognized and do not function in eukaryotic cells. The skilled person can easily determine the most appropriate promoter.

The invention also relates to a genetically engineered DNA molecule comprising a first region encoding a nucleic acid molecule, or RNA molecule, according to the above definition.

A variety of host-vector systems may be utilized to express the RNA molecule, i.e. the aptamer according to the invention. Primarily, the vector system must be compatible with the host cell used. Host-vector systems include but are not limited to the following: bacteria transformed with bacteriophage DNA, plasmid DNA, or cosmid DNA; microorganisms such as yeast containing yeast vectors; mammalian cell systems infected with virus (e.g. vaccinia virus, adenovirus, adeno-associated virus, retrovial vectors, etc.); insect cell systems infected with virus (e.g., baculovirus); and plant cells infected by bacteria or transformed via particle bombardment (i.e., biolistics). The expression elements of these vectors vary in their strength and specificities. Depending upon the host-vector system utilized, any one of a number of suitable transcription elements can be used.

Advantageously, the invention relates to the genetically engineered DNA molecule defined above, further comprising a promoter operably coupled to the first region to control expression of the RNA molecule.

Advantageously, the above defined promoter is a constitutive promoter or an inducible promoter.

The invention further relates to a host cell containing the nucleic acid molecule as defined above, or a molecular complex as defined above, or containing the DNA molecule coding for a nucleic acid molecule as defined above, or the genetically engineered DNA molecule as defined above, or a combination thereof.

Once the constructed DNA molecule has been cloned into an expression system, it is ready to be incorporated into a host cell. Such incorporation can be carried out by the various forms of transformation, depending upon the vector/host cell system such as transformation, transduction, conjugation, mobilization, or electroporation. The DNA sequences are cloned into the vector using standard cloning procedures in the art, as described by Maniatis et al, Cold Springs Harbor, New York (1982)), Suitable host cells include, but are not limited to, bacteria, yeast, mammalian cells, insect cells, plant cells, and the like. The host cell is preferably present either in a cell culture (*ex vivo*) or in a whole living organism (*in vivo*).

Mammalian cells suitable for carrying out the present invention include, without limitation, COS (e.g., ATCC No. CRL 1650 or 1651), BHK (e.g., ATCC No. CRL 6281), CHO (ATCC No. CCL 61), HeLa (e.g., ATCC No. CCL 2), 293 (ATCC No. 1573), CHOP, NS-1 cells, embryonic stem cells, induced pluripotent stem cells, and primary cells recovered directly from a mammalian organism. With regard to primary cells recovered from a mammalian organism, these cells can optionally be reintroduced into the mammal from which they were harvested or into other animals.

Advantageously, the invention relates to the host cell as defined above further containing a fluorophore molecule comprising a methyne bridge between a substituted aromatic ring system and a substituted imidazol(thi)one, oxazol(thi)one, pyrrolin(thi)one, or furan(thi)one, in particular DFHBI.

The invention also relates to a detection array comprising one or more nucleic acid molecules as defined above, tethered to a discrete location on a surface of the array.

The detection array according to the invention is such that the nucleic acid aptamer molecules as defined above are tethered at discrete locations on a substrate surface, i.e., solid support. The solid support used to form the microarray surface can include, without limitation, glass, metal, and ceramic supports. Tethering of the nucleic acid aptamer molecules can be carried out using a 5 ' biotin to streptavidin-coated glass (Arraylt, Inc). Alternatively, the sensor molecules of the present invention can be provided with an extraneous sequence at its 5' end, where the extraneous sequence allows for tethering the sensor molecule to a hybridization partner tethered to the array surface using standard techniques. The hybridization partners can be printed onto the array surface, and the sensor molecules allowed to hybridize prior to or after exposing the sensor to the sample. In these array systems, fluorophore is in solution and is recruited to the glass surface only if the target molecule binds the second domain of the surface-bound aptamer, thereby creating a fluorophore: aptamer :target complex that can be detected, e.g., using TIRF. The sensors can be spotted in an array format, i.e., an array of microspots, or configured in other shapes or designs on surfaces, so that the sensors are positioned in a spatially defined manner. This will allow one or a series of sensors that are specific to distinct target molecules to be assayed following contact with a mixture that contains one or more of the target molecules at known or unknown concentrations. The fluorescence intensity can be used to determine the concentrations if suitable solutions containing known amounts of target analytes are used to calibrate the fluorescence signals.

Detection assays can also be carried out using the aptamer constructs that include a first domain that contains the fluorophore-binding aptamer and a second domain that is a hybridization probe has a nucleotide sequence complementary to a target nucleic acid molecule. For example, to detect viral RNA present in a sample, the hybridization probe will contain a nucleotide sequence complementary to the viral RNA. After attaching any nucleic acid in a sample to a substrate (e.g., glass surface), the sample is exposed to the fiuorophore and the aptamer construct under conditions to allow hybridization to occur. Subsequent detection of the molecular complex (fluorophore: aptamer construct, complementary viral RNA target), as measured by the fluorescent emissions by the fluorophore on the substrate via TIRF, indicates presence of the viral RNA target. This same assay can be carried out using an aptamer construct that possess a second domain, which instead of being a hybridization probe, includes either an aptamer sequence or a non-aptamer sequence that binds to a specific protein (e.g., MS2 sequence binds the MS2 protein or a fusion protein containing the same), in which case binding of the protein to the substrate (e.g., in an ELISA format) will also allow for detection.

Alternatively, detection assays can be carried out using these same types of aptamer constructs using a fixed cell sample or histologic tissue sample.

Where ever the target molecule is present in these samples, the aptamer construct can be bound to the sample and the fluorophore will identify its presence.

The invention relates also to the use of :

the nucleic acid molecule as defined above, or
the molecular complex as defined above, or
the DNA molecule coding for a nucleic acid molecule as defined above, or

the genetically engineered DNA molecule as defined above, or
the host cell as defined above,
or a combination thereof
for imaging, preferably *in vitro* or *ex vivo,* small molecules, RNA and proteins, preferably in cells

The target molecule of interest can be any biomaterial or small molecule including, without limitation, proteins, nucleic acids (RNA or DNA), lipids, oligosaccharides, carbohydrates, small molecules, hormones, cytokines, chemokines, cell signaling molecules, metabolites, organic molecules, and metal ions. The target molecule of interest can be one that is associated with a disease state or pathogen infection.

The invention further relates to a method for imaging, preferably in vitro or ex vivo small molecules, RNA and proteins in cells as defined above, comprising the administration a living in vivo and ex vivo cultures cell a nucleic acid according to the above definition, operably linked to a RNA, along with a fluorophore molecule comprising a methyne bridge between a substituted aromatic ring system and a substituted imidazol(thi)one, oxazol(thi)one, pyrrolin(thi)one, or furan(thi)one, in particular DFHBI.

For instance, for imaging RNA in cells, it is possible to provide to a cell, or to a free-cell expression system, a molecule allowing the expression of a fusion RNA constituted by :

- the RNA to be studied in the cell, operably linked, preferably in its 3'-end, but possibly to its 5'end to
- an aptamer according to the above definition, or to one strand of the aptamer constituted by two separated single stranded molecules.

The above-disclosed fusion RNA is then expressed in the cell, or in a free-cell expression system, and in presence of the fluorophore, the part of the fusion molecule will interact with the fluorophore. This will result in a fluorescence emission upon exposure of an appropriate wavelength, and it could be possible to track, and thus to image, the RNA to be studied, because it is covalently linked to the aptamer.

It would be therefore possible to monitor the trafficking, the localization, the accumulation... of the RNA to be studied, in particular in living cells without alteration of their integrity.

From conventional techniques of molecular biology, the skilled person would be able to obtain all the necessary fusion RNA molecules.

The invention will be better understood from the following figures and in light of the following examples.

**LEGEND TO THE FIGURES**

**Figure 1** represents the effect of monovalent cations (Black : $K^+$; Grey : $Na^+$; hatched : Li+, White : Cs+ and with diamonds : No salt) on the fluorescence of complexes formed between DFHBI and different DFHBI-binding RNA aptamers. 1 $\mu$M of RNA was incubated with 10 $\mu$M DFHBI in 40 mM Tris-HCl, 1 mM $MgCl_2$ and either 100 mM of salt (K, Na, Li or Cs) or no monovalent cation (no salt). The fluorescence of the complex was measured at 25°C. Values are the mean of relative fluorescence of three independent experiments and error bars correspond to $\pm$ 1 standard error. A: Spinach 1.0; B: Spinach 2; C: Broccoli; C: SpiSel and D: iSpinach.

**Figures 2A to 2F** refer to microfluidic-assisted screening.

Figure 2A represents the experimental workflow. Steps performed on-chip (gray boxes) were distinguished from those performed off-chip (white boxes). A. : PCR mixture; B.: Emulsification; C.: Collection and thermocycling; D.: Fusion; E.: Collection and incubation; F.: Fluorescence-activated sorting; G.: improved variants; H.: IVT mixture and I.: Emulsification.

Figure 2B represents the PCR droplets production. Aqueous phase supplemented with a high concentration of an orange fluorescent dye was injected into droplet generator device and 2.5 pL droplets were generated by focusing aqueous (dark gray) and oil (gray) flows. Emulsions were collected and thermocycled.

Figure 2C represents the droplets fusion. Small PCR droplets were reinjected into droplet-fusion device and spaced by a stream of oil. 16 pL droplets containing *In Vitro* Transcription (IVT, light orange) mixture supplemented with DFHBI were concomitantly produced and synchronized with PCR droplets. Pairs of droplets were then fused when passing in between a ground-connected electrode (gnd, in black) and an electrode to which tension (pos, light grey) was applied.

Figure 2D represents the droplets sorting. After incubation, emulsions were reinjected into a Fluorescence Activated Droplet Sorting device and the fluorescence of each droplet read at a detection point (arrow and black line on the micrograph). Based on the fluorescence signal, droplets of interest (medium grey) were deflected into sort channel (up) by applying tension to one of the electrode (pos) whereas non-fluorescent droplets (dark grey) flowed into the waste channel (down).

Figure 2E represents a typical fluorescence profile of screened emulsion. The analysis of DFHBI green fluorescence and Texas-Red orange fluorescence allowed identifying the different populations composing the emulsion. Indeed, using orange fluorescence signal, IVT droplets fused to single PCR droplets (populations B to E) were easily discriminated from unfused (population A) and double fused (population F) IVT droplets. Green fluorescence resulting from EvaGreen intercalation allowed discriminating droplets containing amplified DNA (population D) from droplet resulting from fusion with an initially empty PCR droplet (population B). Finally, the stronger DFHBI green fluorescence allowed discriminating droplets containing non-fluorogenic (population B) and highly fluorogenic aptamers (population E, dashed boxed). Population E was gated and corresponding droplets sorted. A represent 3%; B represents 77.5%; C represents 2.2%; D represents 12%; E represents less than 1.5% and F represents 3.6% of the studied population. Y-axis: Orange fluorescence (RFU) and x-axis : Green fluorescence (FU). N=130,000 events.

Figure 2F represents a graph showing the evolution profiles of SpiSel-derived mutants. Gene libraries obtained after mutagenesis (in grey) or screening steps (in white) were transcribed, the RNAs purified and their fluorogenic properties assayed in the presence of the salt used for the selection. Fluorescence values were normalized to that of SpiSel (black circle) in the same conditions. Screenings performed in potassium (triangles) were distinguished from those performed in sodium (squares). Values are the mean of two independent experiments and error bars correspond to ± 1 standard error. Y axis : relative fluorescence; x-axis: round of mutagenesis (M)/Screening (S).

**Figures 3A to 3D** represent the engineering of improved aptamers.

Figure 3A represents the secondary structure models of aptamers. Molecules are represented according to the model introduced in (21). Mutations are shown with a circle and numbering adapted to molecule length. From left to right: 4.68 aptamer; 4-68-J1/2 aptamer; 4-68/49 aptamer and min68/49-3 aptamer. Q= Quartet.

Figure 3B represents the effect of J1/2 deletion and A42U mutation on the fluorescence of aptamer 4-68. A.: 4-68-J1/2; B. 4-68/49. Grey column represents Na; Dark grey column represents K. Y-axis represents the relative fluorescence to 4-68.

Figure 3C represents effect of min68/49 miniaturization on complex fluorescence in the presence or absence of constant regions. Mutant min68/49 was shortened by 3 (-3) or 6 (-6) base pairs. The fluorescence of constructs was tested in the presence and absence of constant regions. Grey column represents Na; Dark grey column represents K. A.: + constant region, B.: - constant region. Y-axis represents the relative fluorescence to 4-68/69.

Figure 3D represents effect of double mutations on min68/49-3 fluorescence. The nature of the monovalent cation used is indicated by the color code shown in the inset. Assays were performed with 1 μM RNA and 10 μM DFHBI at 37°C. Values are the mean of three independent experiments and error bars correspond to ± 1 standard error. Grey column represents Na; Dark grey column represents K, hatched columns represents Li and white column represents Cs. A.: + min68/49-3 G14A/G56A, B.: - min68/49-3 U31A/C32U. Y-axis represents the relative fluorescence to min68/49-3.

**Figure 4** represents a comparison of Spinach2 and iSpinach. The secondary structure models of the RNAs were represented according to the model introduced in (21): left: Spinach 2.0 ; right : iSpinach. Mutations are shown with a circle and numbering adapted to molecule length. Below the structures, the table represents the aptamers properties. Main parameters including Folding Efficiency (F.E.), melting temperature of the complex ($T_m^{cplx}$) and dissociation constant ($K_d$) of each RNA as well as the Relative Molar Fluorescence of DFHBI were determined at 25°C. Values are the mean of three independent experiments and error bars correspond to ± 1 standard error. Excitation/emission spectra are shown on **Figure 9**. Q= Quartet.

**Figures 5A to 5C** represent the real-time monitoring of RNA synthesis and ribozyme activity.

Figure 5A represents the constructs used. DNA fragments coding for iSpinach, Spinach2 or iXm1 ribozyme were combined in different ways and placed under the control of T7 RNA polymerase promoter sequence ($Pro_{T7}$).

Figure 5B represents the real-time ribozyme activity monitoring. The different constructs were *in vitro* transcribed in the presence of ribozyme fluorogenic substrate (S21-Atto) and the fluorescence monitored at 37°C. Product generation rate ($\frac{dP}{dt}$) and uncatalyzed reaction rate ($k_{uncat}$) were determined as the slope of the linear phase of reaction respectively in the presence and absence of ribozyme. $\frac{dP}{dt}$=0.93 pmol/min and $k_{uncat}$ = 0.17 pmol/min. Y-axis represents the generated products (pmol) and x-axis represents the time in minutes. White circle; blank; grey square : iXmn1-iSpinach; black diamonds : iXmn1-Spinach 2; grey triangle : iXmn1; white square : iSpinach and white diamond : Spinach 2.

Figure 5C represents the real-time transcription monitoring. The *in vitro* transcription mixture used in B. was supplemented with DFHBI (instead of S21-Atto) and the fluorescence monitored at 37°C. The synthesis rate ($\sigma$) was determined as the slope of the linear phase. $\sigma_{\text{iXmn1-iSpinach}}$ = 2.1pmol/min and $\sigma_{\text{iXmn1-Spinach}}$ = 1.5 pmol/min. Y-axis represents RNA synthetized (pmol) and x-axis represents the time in minutes. White circle; blank; grey square : iXmn1-iSpinach; black diamonds : iXmn1-Spinach 2; grey triangle : iXmn1; white square : iSpinach and white diamond : Spinach 2.

**Figure 6** represents the fluorescence of isolated mutants. Aptamer-coding genes were PCR amplified and DNA in vitro transcribed in the presence of DFHBI. The cotranscriptional fluorescence increase was monitored at 37°C. Relative fluorescence was calculated by dividing the fluorescence increase rate of the analyzed clone by the one of SpiSel. Y-axis represents the relative fluorescence.

**Figures 7A and 7B** represent the mutations isolated from the in vitro evolution process and summary of the changes used to convert Spinach 1.1 into iSpinach.

Figure 7A represents the mutations isolated for the screening and identified through sequence alignments (Table S3) are shown between brackets and mapped onto the secondary structure of the original Spinach 1.1.

Figure 7B represents the conversion of Spinach 1.1 into iSpinach. The mutations originally identified from the screening process are shown in grey, the residue rationally transplanted is squared and the nucleotides rationally deleted are shown at position.

**Figures 8A and 8B** represent the effect of the temperature on RNA/DFHBI complex dissociation. Arrehnius plots are shown for DFHBI in complex with Spinach2 (Figure 8A) or iSpinach (Figure 8B) in the presence of potassium (squares) or sodium (triangles). Y-axis represents $\ln(K_d)$ and x-axis represents 1/T (K x $10^{-3}$).

**Figure 9** represents the excitation and emission spectra of DFHBI in complex with Spinach2 or iSpinach in the presence of sodium or potassium. Excitations spectra (dashed lines) were determined by recording the fluorescence emitted at 500 nm following excitation at a wavelength ranging from 350 nm to 400 nm with a step of 1 nm. Emission spectra (continuous line) were determined by exciting the sample at 400 nm and recording the fluorescence emitted from 475 nm to 600 nm with a step of 1 nm. Spinach and iSpinach spectra in potassium are similar whereas iSpinach spectra in sodium have an increased wavelength. Y-axis represents the normalized excitation and emission and x-axis represents the wavelength in nm.

**Figure 10** represents a graph showing iSpinach (B) and Spinach2 (A) fluorescence in extracellular environment. 1$\mu$M of RNA was incubated with 10 $\mu$M DFHBI in HL3 medium and the green fluorescence was recorded at 25°C (white columns), 37°C (black columns) or 45°C (hatched columns). Y-axis represent the green fluorescence in RFU.

**Figures 11A to 11D** represent photography showing in vivo expression of iSpinach in bacteria.

Figure 11A is a bright-field microscopy photography of bacteria expressing an aptamer not able to interact with DFHBI.

Figure 11B is a fluorescence-microscopy photography of bacteria expressing an aptamer not able to interact with DFHBI.

Figure 11C is a bright-field microscopy photography of bacteria expressing iSpinach.

Figure 11D is a fluorescence-microscopy photography microscopy photography of bacteria expressing iSpinach.

## EXAMPLES

### Example 1 - Characterization of iSpinach

Using random mutagenesis and high throughput screening by microfluidic-assisted In Vitro Compartmentalization, the inventors report the isolation of an order of magnitude times brighter mutants of the light-up RNA aptamers Spinach that are far less salt-sensitive and with a much higher thermal stability than the parent molecule. Further engineering gave iSpinach, a molecule with folding and fluorescence properties surpassing those of all currently known aptamer based on the fluorogenic co-factor 3,5-difluoro-4-hydroxybenzylidene imidazolinone (DFHBI). The inventors illustrate the potential of iSpinach in a new sensitive and high throughput-compatible fluorogenic assay that measures co-transcriptionally the catalytic constant (kcat) of a model ribozyme.

In the example presented herein, the inventors show that none of the DFHBI-binding aptamers currently known was optimal for *in vitro* applications since the fluorescence of the complex they generate with DFHBI had a limited thermal stability and strongly depended on the nature of the salt present in the reaction mixture. Using the microfluidic-assisted *In Vitro* Compartmentalization (μIVC) procedure the inventors recently introduced (29) allowed them to screen Spinach gene libraries while applying selection pressures favoring the isolation of aptamers able to work at high temperature and in a potassium free environment. Identified beneficial mutations were combined and the molecule further engineered to afford iSpinach, an aptamer with properties surpassing those of all DFHBI-binding aptamer described so far. Finally, the inventors demonstrate the potent value of this new aptamer by setting-up a sensitive and high throughput-compatible fluorogenic assay able to assess co-transcriptionally the catalytic constant ($k_{cat}$) of a model ribozyme.

**Material and methods**

**Gene library generation.** The sequence coding for Spinach 1.1 (19) was flanked with constant regions at 5' (GGAAGACGTAGCAAG - SEQ ID NO: 23 ) and 3' ends (CAGAGAGATGACAGAGAACACA- SEQ ID NO: 24) to afford SpiSel. Mutant libraries were generated by error-prone PCR by subjecting 0,04 ng of DNA to 4 amplification cycles in the presence of Rev (TGTGTTCTCTGTCATCTCTCTG - SEQ ID NO: 25) and Fwd (GTAAAACGACGGCCAGTGCCAAGCTTGCATGCTAATACGACTCACTATAG GAAGACGTAGCAAG - SEQ ID NO: 26) primers as well as nucleotide analogues (JBS dNTP-Mutagenesis Kit, Jena Bioscience) as described before (29). 1 ng of PCR products was amplified in a second PCR mixture containing 10 pmol of each primer (Fwd and Rev), 0.2 mM of each dNTPs, 5 U of DreamTaq™ and the corresponding buffer (Fermentas). The mixture was thermocycled starting with an initial step of denaturation of 2 min at 92°C followed by 4 cycles of: 1 min at 92°C, 1 min at 55°C, and 5 min at 72°C, and PCR products were purified. The starting library was TA-cloned the sequence of 26 clones revealed an average mutation rate of 4.23 mutation per variant.

**TA-cloning and sequencing.** Genes contained in mutant libraries were diluted in a mixture containing 10 pmol of each primer (Fwd and Rev), 0.2 mM of each dNTPs, 5 U of DreamTaq™ and the corresponding buffer (Fermentas). The mixture was then subjected to an initial denaturation step of 3 min at 95°C, followed by 25 cycles of: 30 sec at 95 °C, 30 sec at 60 °C, and a final extension step of 10 min at 72°C. PCR products were inserted in pTZ57R/T vector following manufacturer's instruction (InsTAclone PCR cloning Kit, Thermo-Scientific). Ligation products were recovered by phenol/chloroform extraction and 100 ng of DNA used to transform Electro-10 blue bacteria (Agilent) placed in 2mm electroporation (MicroPulser, Bio-Rad). After an hour of recovery at 37°C under agitation, bacteria were plated on Luria broth (LB)-Amplicillin agar plate and incubated overnight at 37 °C. The colonies were picked, used to inoculate liquid LB and grown at 37°C until saturation. Plasmids DNA were extracted using "GenJet Plasmid Miniprep kit" (Thermo-Scientific), and sequences determined by Sanger approach (GATC Biotech).

**Microfluidic chip fabrication and manipulation.** The description of the microfluidic devices, their fabrication as well as the microfluidic workstation used in this work can be found in (29).

**Microfluidic-assisted *In vitro* Compartmentalization screening**

**i/Digital Droplet PCR.** DNA mutant libraries were diluted in 200 μg/mL yeast total RNA solution (Ambion) down to ~8 template DNA molecules per picoliter. 1 μL of this dilution were then introduced in 100 μL of PCR mixture containing 20 pmol of each primer (Fwd and Rev), 0.2 mM of each dNTPs, 0.67 mg/mL Dextran-Texas Red 70 kDa (Molecular Probes), 0,1 % Pluronic F68, 1x EvaGreen (Biotium), 5 U of DreamTaq™ and the corresponding buffer (Fermentas). The mixture was loaded in a length of PTFE tubing and infused into droplet generator microfluidic chip where it was dispersed in 2.5 pL droplets (production rate of -12,000 droplets/second) carried by HFE 7500 fluorinated oil (3M) supplemented with 3% of a fluorosurfactant (29). Droplet production frequency were monitored and used to determined droplet volume, and pumps flow rates (MFCS, Fluigent) adjusted to generate 2.5 pL droplets. Emulsions were collected in 0.2 μL tubes as described before (29) and subjected to an initial denaturation step of 1 min at 95°C followed by 30 cycles of: 1 min at 95°C, 1 min at 55°C, 2 min at 72°C. Droplets were then reinjected into a droplet fusion microfluidic device.

**ii/ Droplet fusion.** PCR droplets were reinjected and spaced into fusion device at a rate of -1,500 droplets/second. Measuring the fraction of droplets with an increased EvaGreen fluorescence allowed assessing droplet occupancy. Each PCR droplet was then synchronized with a 16 pL *in vitro* transcription (IVT) droplet containing 2.2 mM of each NTP (Larova), 24 mM $MgCl_2$, 44 mM Tris-HCl pH 8.0, 5 mM DTT, 1 mM Spermidine, 35 μg/mL of Dextran-Texas Red 70 kDa (Molecular Probes), 0,1% Pluronic F68, 20 μg/mL T7 RNA polymerase, 200 μM DFHBI (Lucerna), 1 μg inorganic pyrophosphatase (Roche) and 50 mM NaCl or KCl. IVT mixture was loaded in a length of PTFE tubing kept on ice during all experiment. PCR droplets were spaced and IVT droplets produced using a single stream HFE 7500 fluorinated oil (3M) supplemented with 2 % (w/w) of fluorinated. Flow-rates (MFCS, Fluigent) were adjusted to generate 16 pL IVT droplets and maximize synchronization of 1 PCR droplet with 1 IVT droplet. Pairs of droplets were then fused with an AC field (350 V at 30 kHz) and the resulting emulsion collected off-chip and incubated for 2 hours at 37°C.

**iii/ Droplet analysis and sort**

The emulsion was finally reinjected into of analysis and sorting microfluidic device mounted on Thermo plate (Tokai Hit) holding the temperature at 45°C. Prior to starting droplet fluorescence analysis and sort, the temperature of the microfluidic device was allowed to equilibrate at 45°C for 10 minutes. The proper warming of the chip controlled with a thermocouple. The micrometric depth of the channels together with the low droplets re-injection flow rate used were expected to allow equilibration of droplets temperature to that of the device prior to analyzing their fluorescence. Droplets were reinjected at a frequency of ~200 droplets/second and spaced with a stream of surfactant-free HFE 7500 fluorinated oil (3M). The green fluorescence (DFHBI complexed with the aptamer) of each droplet was analyzed and the 1 to 2 % most green fluorescence droplets were sorted. The gated droplets were deflected into collecting channel by applying a 1 ms AC fields (1200 V 30 kHz) and collected into a 1.5 mL tube. Residual droplets were recovered from the collection tubing by flushing 200 µL of HFE fluorinated oil (3M). 100 µL of 1H, 1H, 2H, 2H-perfluoro-1-octanol (Sigma-Aldrich) and 200 µL of 200 µg/mL yeast total RNA solution (Ambion) were then added and droplets broken by vortexing the mixture. DNA-containing aqueous phase was then recovered.

**Enrichment measurement and colony screening.** Genes contained in 2 µL of DNA-containing aqueous phase from unsorted or sorted fractions were placed in 100µL of PCR mixture identical to that used for TA-cloning and thermocycled starting with an initial cycle of 30sec at 95°C, followed by 25 cycles of: 5 sec at 95°C and 30 sec at 60°C. Then 2.5 µL of this PCR product were incubated with 16 µL of IVT mixture identical to that used for the µIVC screening containing 200 µM DFHBI but deprived of dextran-Texas Red and pluronic. The transcription mixture was then incubated at 37°C in a real-time thermocycler (Mx 3005P, Agilent) and the green fluorescence (ex: 492 nm/em: 516 nm) measured every minute for 2h.

Enriched libraries from the last round of screening in the presence of sodium or potassium were TA-cloned. 90 clones were randomly picked, genes coding for the DFHBI-binding aptamers were PCR-amplified and *in vitro* transcribed as for enrichment measurement. Following an hour of transcription real-time monitoring at 37°C, the mutants with the highest apparent transcription rate were sequenced.

**RNA synthesis and purification.** Genes coding for aptamers of interest were PCR amplified with the same procedure used for enrichment measurements. 20 µL of PCR products were then *in vitro* transcribed in 230 µL of mixture containing 2 mM of each NTP, 25 mM $MgCl_2$, 40 mM Tris-HCl pH 8.0, 5 mM DTT, 1 mM Spermidine and 70 µg/mL T7 RNA polymerase. After 4h of incubation at 37°C, 10 units of Baseline-Zero™ DNase (Epicentre) and the corresponding buffer were added to the mixture and a second incubation of 1h at 37°C performed. RNAs was recovered by phenol extraction followed by an ethanol precipitation in the presence of 300 mM sodium acetate pH 5.5 (Sigma-Aldrich). After centrifugation and a wash in 70% ethanol, the pellets were dissolved in denaturing buffer (0.05% bromophenol blue, 0.05% xylene cyanol, 20% glycerol, TBE 1x, 8M urea) and the solution loaded on a 12% denaturing 8 M urea acrylamide/bisacrylamide gel. The piece of gel containing RNA was identified by UV shadowing, sliced from the gel and transferred in a dialyze tube (MWCO = 3 500, Spectrum Lab) filled with TBE. RNA was electro-eluted by placing the montage in TBE for 1h00 at 100 V. Eluted RNA were filtered in centrifuge tube 0,45 µm (VWR) and ethanol precipitated in the presence of 300 mM sodium acetate pH 5.5. After centrifugation and a wash in 70% ethanol, the pellets were dissolved in DEPC-Treated water and quantified with Nanodrop (Thermo Scientific).

**Fluorescence measurement of purified RNA.** A 2 µM of purified RNA was heated for 2 min at 85°C and cooled at 25°C for 5 min. The solution was next mixed with 1 volume of a mixture containing 80 mM Tris-HCl pH 7.5, 2 mM $MgCl_2$, 20 µM DFHBI, 200 mM of salt (KCl, NaCl, LiCl or CsCl). The mixture was then incubated at 25°C for 15 min prior to fluorescence measurement. Green fluorescence (ex: 492 nm/em: 516 nm) was then monitored for 5 min at 25°C on a real-time thermocycler (Mx 3005P, Agilent). For $T_m^{app}$ measurement, the temperature was gradually increased from 25 to 60°C with a step of 5°C and a 5 min fluorescence monitoring was performed at each temperature.

**Folding Efficiency.**

Folding Efficiency was determined using procedure described in (19). The green fluorescence was measured (ex: 492 nm/em: 516 nm) at three temperatures (25°C, 37°C, 45°C) on a real-time thermocycler (Mx 3005P, Agilent).

**Affinity measurements.**

To measure $K_d$, the concentration of DFHBI was progressively increased from 100 nM to 200 µM with 100 nM of renatured RNA in 40 mM Tris-HCL pH 7.5, 1 mM $MgCl_2$, 100 mM NaCl or KCl and 2,5% DMSO). The fluorescence was measured (ex: 492 nm/em: 516 nm) at three temperatures (25°C, 37°C, 45°C) on a real-time thermocycler (Mx 3005P, Agilent).

**Co-transcriptional $k_{cat}^{app}$ measurement.**

0.6 pmol of DNA bearing T7 promoter were introduced in 60 µl of mixture containing 40 mM Tris-HCl pH 8.0, 22 mM

$MgCl_2$, 5 mM DTT, 1 mM Spermidine, 1,6 mM of each NTP and 65 U/μL T7 RNA polymerase (NEB). 200 pmol of DFHBI or 20 pmol of S21-Atto(29) were then added to 20 μL aliquots and the green fluorescence (ex: 492 nm/em: 516 nm) was monitored for 1h00 at 37°C in a real-time thermocycler (Mx 3005P, Agilent). Fluorescence values were converted into pmol of generated product or synthesized RNA using experimentally measured molar fluorescence of S21-Atto and DFHBI respectively.

**Spectral analysis.**

Absorbance was realized on UV-Vis spectrophotometer (Agilent Technology) fom 350 nm to 500 nm. Emission (from 410 nm to 60 nm) and excitation (from 350 to 490 nm) was recorded by FluoroMax-4 spectrofluorometer (Horiba Scientific). This measurement was realized in condition with excess RNA.

**Results and discussion**

**Evaluation of DFHBI-binding aptamers performances *in vitro***

The inventors started their study by characterizing the performances of the three DFHBI-binding aptamers described so far: Spinach1.0, Spinach2 and Broccoli (4,6,19). To assess unambiguously the intrinsic fluorescence and the folding properties of these molecules, aptamers were used in their minimal version instead of being inserted into a tRNA scaffold (30) as in previously reported (4,19). Coding-genes were *in vitro* transcribed, RNA were gel-purified and renaturated just prior to being used.

First, 10 pmol of aptamer (1 μM) were incubated with 1 nmol of DFHBI in a buffer mimicking *in vivo* conditions (40 mM Tris-HCl pH 7.5, 1 mM $MgCl_2$, 100 mM KCl) and the fluorescence of DFHBI/RNA complexes was measured. As expected from a previous report (19) Spinach2 was twice more fluorescent than Spinach 1.0 in the present conditions (**Figure 1**). Surprisingly, Broccoli, a variant with superior properties *in vivo* (4), generated the dimmest complex with DFHBI *in vitro* (**Figure 1**). This bad performance was partly explained by a low folding efficiency (F.E. < 18 % at 25°C, see

**Table 1)** of the aptamer in these conditions.

| | 25°C | | 37°C | | 45°C | |
|---|---|---|---|---|---|---|
| | $K^+$ | $Na^+$ | $K^+$ | $Na^+$ | $K^+$ | $Na^+$ |
| Spinach 2 | 61.0 ±12.2 | n.m. | 27.0 ± 4.7 | n.m. | 2.3 ± 1.4 | n.m. |
| Broccoli | 17.7 ± 3.2 | n.m. | 15.5 ± 4.8 | n.m. | 9.9 ± 2.6 | n.m. |
| iSpinach | 69.0 ± 4.0 | 59.6 ± 8.1 | 53.7 ± 3.7 | 23.6 ± 3.4 | 37.4 ± 3.2 | 6.4 ±1.5 |

Table 1 represents the folding efficiency of Spinach2, Broccoli and iSpinach as a function of temperature in the presence of sodium or potassium. Values are expressed in percentage of folded molecule and are the mean of three independent experiments and error bars correspond to ± 1 standard error. n.m. : not measurable.

A lower F.E. of Broccoli compared to Spinach2 was already suggested in a previous work in which such a strong difference was probably minimized as the aptamer was inserted into a tRNA scaffold that assisted aptamer folding (4). Consequently to this low fluorescence *in vitro,* Broccoli was no longer considered in the rest of the study.

The inventors next investigated the effect of exchanging potassium for another monovalent ion on the fluorescence of DFHBI in complex with Spinach1.0 or 2. Indeed, whereas potassium is the main monovalent cation found *in cellulo* (31), it might not be dominant ions in the extracellular environment and even be absent from *in vitro* reaction mixtures. Using 100 mM NaCl instead of KCl in the reaction mixture led to a significant drop of the fluorescence of both Spinach variants (**Figure 1**) confirming data previously obtained with Spinach (20). Whereas a residual fluorescence was still observed with sodium, the use of lithium, cesium or the lack of monovalent cation completely abolished the fluorescence of Spinach aptamers (**Figure 1**). The differential effect of the cations confirmed that proper stabilization of the G-quadruplex motif of the aptamer was necessary for efficient recognition of DFHBI and that the fluorescence of DFHBI/aptamer complex strongly depends on the nature of the monovalent cation present.

Beside the strong dependence of Spinach 1.0 and 2 on potassium, both molecules were also reported to have a limited thermal stability as the complexes they generate with DFHBI have melting temperature of ~34°C and ~38°C respectively (19). Accordingly, the inventors found that, in the presence of potassium, the fraction of Spinach2 aptamer properly folded decreased from 61% to 27% at 25°C and 37°C respectively (**Table 1** above).

Overall, these different observations show that none of the DFHBI-binding aptamer described so far was optimized for *in vitro* application (e.g. at 37°C in a reaction mixture not supplemented with potassium). The folding efficiency of the RNA was the main limiting parameter, suggesting that re-evolving the molecule *in vitro* by applying selection pressures favoring the isolation of a thermostable fluorogenic aptamer in a potassium-free environment should lead to the identification of a mutant with properties surpassing those of the rationally designed Spinach2. The use of such conditions that destabilize the molecular folding has already proved to be an efficient strategy as it allowed, for instance, the identification of the Superfolder GFP (32).

## Improvement of Spinach properties by *in vitro* evolution

Spinach1.1 was used as starting point of the evolutionary process as its sequence differed from the best variant isolated from SELEX only by a single stabilizing mutation in the basal stem (19). Constant regions were appended at the 5' of the molecule to ensure a homogeneous transcription initiation and at its 3' end to enable PCR amplification of the gene. The resulting molecule (SpiSel) was found to have a fluorescence slightly improved (**Figure 1**). Mutant libraries were then generated by error-prone PCR using a mutation rate of ~4 mutations per gene and subjected to several rounds of high throughput screening using a procedure of microfluidic-assisted *In Vitro* Compartmentalization ($\mu$IVC) the inventors recently developed (29). Even though, this method has a lower throughput than conventional SELEX, it has the great advantage of directly screening libraries for mutants able to generate a fluorescent complex with DFHBI.

Each round of $\mu$IVC selection comprised 3 main steps (**Figure 2**). First, the DNA library was diluted into a PCR mixture down to a concentration of 1 molecule/12.5 pL and the aqueous phase was dispersed into highly monodisperse 2.5 pL droplets carried by a fluorinated oil phase using a first microfluidic device (**Figure 2B**). The used dilution allowed having, on average, -0.2 molecule of DNA per droplet to maximize droplet occupancy (~18%, **Table 2**) while limiting multiple encapsulation events (< 10% of the occupied droplets contained more than 1 gene).

Table 2 represents the experimental parameters. $\lambda$ value were calculates as described in (29)

| Salt | Round | PCR droplets occupancy (%) | $\lambda$ value | Fusion efficiency (%) | Number of analyses droplets | Number of sorted droplets |
|---|---|---|---|---|---|---|
| Sodium | S1 | 32 | 0.38 | 92 | 1,392,500 | 9,028 |
| | S2 | 12 | 0.12 | 75 | 250,950 | 2,526 |
| | S3 | 15 | 0.16 | 85 | 776,251 | 11,278 |
| | S4 | 16 | 0.17 | 90 | 879,000 | 7,896 |
| Potassium | S1 | 26 | 0.30 | 70 | 738,875 | 22,730 |
| | S2 | 23 | 0.26 | 87 | 397,950 | 4,514 |
| | S3 | 17 | 0.18 | 80 | 1,526,750 | 28,071 |
| | S4 | 18 | 0.19 | 75 | 225,750 | 2,589 |

Droplets production monitoring was possible by supplementing the PCR mixture with a high concentration of an orange fluorescent dye (670 $\mu$g/mL Dextran-Texas Red). The emulsion was collected and thermocycled to amplify the DNA prior to reinjecting the droplets into a second microfluidic chip (**Figure 2C**) where each small droplet was synchronized and fused with an on-chip generated 16 pL droplet containing an *In Vitro* Transcription (IVT) mixture supplemented with 200 $\mu$M DFHBI, 50 mM NaCl and a low concentration of the orange fluorescent dye (35 $\mu$g/mL Dextran-Texas Red). The new emulsion was collected and incubated. The different concentrations of orange fluorescent dye in PCR and IVT droplets allowed discriminating unfused IVT droplet (low orange fluorescent population 1 on **Figure 2E**) from IVT droplets fused with 1 (populations 2 to 5 on **Figure 2E**) or 2 PCR droplets (population 6 on **Figure 2E**) and to adjust the flow parameters to maximize the fusion of 1 IVT droplet to 1 PCR droplet (70 to 92%, **Table 2** below). On another hand, the use of sodium was expected to weaken the G-quadruplex structure while maintaining a residual fluorescence allowing for detecting positive droplets. This was used as a selection pressure favoring the isolation of mutants with improved folding properties. Finally, the emulsion was reinjected into a droplet sorting microfluidic device (**Figure 2D**) warmed at 45°C, a selection pressure expected to favor the isolation of RNA with improved thermal stability. The orange and green fluorescence of each droplet was measured and the 1% most green fluorescent droplets of the single fused population (population 5 on **Figure 2E**) were recovered at the end of each round (**Table 2 below**). To summarize, three selection pressures (use of sodium, sorting device warming and selection of the DFHBI/aptamer complexes based on their brightness) were simultaneously applied to favor the isolation of DFHBI-binding aptamers able to fold efficiently and to form a thermostable fluorescent complex with DFHBI.

A total of 2 pairs of rounds of selection, interspersed by a round of mutagenesis to maintain genetic diversity, were performed and allowed increasing the average molar fluorescence of the population -3.5 times (gray triangles on **Figure 2.F**) with respect to the starting molecule (SpiSel). Interestingly, performing the same *in vitro* evolution experiment using KCl instead of NaCl did not significantly improved the fluorescence of the population after 4 rounds of selection (gray squares on **Figure 2.F**), confirming that sodium was an efficient selection pressure. The enriched library from the fourth round of selection in sodium was then cloned and the fluorescence of 85 individual mutants was analyzed (**Figure 6**). More than 95 % of the tested clones were more fluorescent than SpiSel. Whereas the majority of these clones were 2 to 6 times more fluorescent than SpiSel, the variant 4-68 was surprisingly ~10 times more fluorescent (**Figure 6**). Sequence analysis (**Table 3** and **Figures 7**) revealed that 4-68 differed from SpiSel by 8 mutations (**Figure 3A**).

| Position[a] | 16 | 17 | 18 | 19 | 20 | 21 | 22 | 23 | 24 | 25 | 26 | 27 | 28 | 29 | 30 | 31 | 32 | 33 | 34 | 35 | 36 | 37 | 38 | 39 |
|---|---|---|---|---|---|---|---|---|---|---|---|---|---|---|---|---|---|---|---|---|---|---|---|---|
| Region[b] | P1 | | | | | | | | | J1-2 | | | | | | | | P2 | | | | | | |
| SpiSel | G | A | C | G | C | G | A | C | U | G | A | A | U | G | A | A | A | U | G | G | U | G | A | A |
| Clone68 | | | U | | | | | | | | | G | | | | | | C | | | | | | G |
| Clone34 | x | C | | | | | | U | | | | C | | | | | | | | | | | | G |
| Clone49 | | x | | | U | | | | | | | | | | | | | | | | | | | |
| Clone56 | | x | x | | | | | | | | | | | | | | | C | | | | | | |
| Clone46 | x | x | x | x | | | | | | | | | | | | U | | | | | | | | |
| Clone70 | | | U | | | | | | | | | | | | | | | C | | | | | | |
| Clone41 | | | U | | | | | | | | | | | | | | | C | | | | | | |
| Clone84 | | | U | | | | | | | | G | | | | | | | | | | A | | | |
| Clone79 | | x | U | | | | | | | | | | | | | | | C | | | | | | |
| Clone62 | | | | | | | | | | | | | | | | | | | | | | | | |
| Clone24 | A | | | | | | | | | | | | | | x | x | x | | x | | | | | |
| Clone77 | | | | | | | | | | | | | | | | | | | | | | | | |
| Clone86 | | | | | | | | | | | | G | | | | | | C | | | | | | |
| Clone51 | | | | | | | G | | | | | | | | | | | C | | | | | | |
| Clone73 | | | | | | | | | | | G | | | | | | | C | | | | | | |
| Clone78 | | | U | | | | | | | | | | | | | | | | | | | | | |
| Clone45 | | | U | | | | | | | | | | | | | | | C | | | | | | |
| Clone67 | | | | | | | | | | | | | | | | | | C | | | | | | |
| Clone65 | | | | | | | | | C | | x | x | x | | | G | | C | | | | | | |
| Clone63 | | | U | | | | | | | | | x | x | | x | x | x | | | | | | | |
| Clone50 | | | | | | | | A | | | | | x | | x | | | | | | | | | |
| Clone39 | | | | A | | | | U | | | | | | | | | | C | | | | | | |
| Clone50 | | | | | | | | A | | | | | x | | x | | | | | | | | | |
| Clone64 | | G | | | | | | | | | | G | | | x | x | | | | | | | | |
| Clone22 | | G | U | | | | | | | | | G | | | | U | | | | | | | | |
| Clone23 | | | U | | | | | | | | | U | | | | | | C | | | | | | G |

| Position[a] | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 |
|---|---|---|---|---|---|---|---|---|---|---|---|---|---|---|---|---|---|---|---|---|---|---|---|---|---|---|
| Region[b] | Quartet | | | | | | P3 | | | | | | | | | | | | | | | | L3 | | | |
| SpiSel | G | G | A | C | G | G | G | U | C | C | A | G | G | U | G | U | G | G | C | U | G | C | U | U | C | G |
| Clone68 | | | | | | | | | | | | | | | | | | | | | | U | C | | | |
| Clone34 | | | | | | | | | | | | | | | A | | | | | | | | | | | |
| Clone49 | | | U | | | | | | | | | | | | | | | | | | | | | | | |
| Clone56 | | | | | | | | | | | | | | C | | | A | | | | | | C | | | |
| Clone46 | | | | | | | | | | | | A | | | | | | | | | | | | | | |
| Clone70 | | | | | | | | | | | | | | | A | | | | | | | | | C | U | |
| Clone41 | | | | | | | | | | | | A | | | | | | | | | | | | | | |
| Clone84 | | | | | | | | | | | | | | | | | | A | | | | U | | | | |
| Clone79 | | | | | | | | | | | | | | | | C | | | | | | | | | | |
| Clone62 | | | | | | | | | | | | | | C | U | | | | | | | | | | | |
| Clone24 | | | | | | | | | | | | | | | | | | | | | | | | | | |
| Clone77 | | | | | | | | | | | | | | | A | | | | | | | | C | | | |
| Clone86 | | | | | | | | | | | | A | | | | | | | | | | | | | | |
| Clone51 | | | | | | | | | | | | | | | A | | | A | | | | | | | | |
| Clone73 | | | | | | | | | | | | A | | | | | | U | | | | | | | | A |
| Clone78 | | | | | | | | | | | | | | | C | | | | | | | | | | | |
| Clone45 | | | | | | | | | | | | | | | | | | A | | | | | | | | |
| Clone67 | | | | | | | | | | | | | | | | | | | | | | U | | | | |
| Clone65 | | | | | | | | | | | | | | | | | | | | | | | C | | | |
| Clone63 | | | | | | | | | | | | A | | | | | | | | | | | | | | |
| Clone50 | | | | | | | | | | | | | | | | | | | | | | | | | | |
| Clone39 | | | | | | | | | | | | | | | | | | | | | | | C | | | |
| Clone50 | | | | | | | | | | | | | | | | | | | | | | | | | | |
| Clone64 | | | | | | | | | | | | | | | | | | | | | | | | | | |
| Clone22 | | | | | | | | | | | | | A | | | | | | | | | | | | | |
| Clone23 | | | | | | | | | | | | | | | | | | | | | | | | | | |

Table 3- part 1 represents the sequences of isolated mutants. Black crosses represent deletions.

[a] Positions numbered according to SpiSel sequence

[b] Regions labeled according to the model proposed in (2)

| Position[a] | 66 | 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 |
|---|---|---|---|---|---|---|---|---|---|---|---|---|---|---|---|---|---|---|---|---|---|---|---|---|
| Region[b] | P3 | | | | | | | | | | | | | | | | | Quartet | | | | | | |
| SpiSel | G | C | A | G | U | G | C | A | G | C | U | U | G | U | U | G | A | G | U | A | G | A | G | U |
| Clone68 | | | | | | | | | | | C | | | | | | | | | | | | | |
| Clone34 | | | | | | | | | A | | | | | | | | | | | | | | | |
| Clone49 | | | | | | | | | | | | | | | | | | | | | | | | |
| Clone56 | | | | | | | | | | | | | | | | | | | | | | | | |
| Clone46 | | | | | | | | | | | | | | | | | | | | | | | | |
| Clone70 | | | | | | A | | | | | C | | | | | | | | | | | | | |
| Clone41 | | | | | | A | | | | | | | | | | | | | | | | | | |
| Clone84 | | | | | | A | | | | | | | | | | | | | | | | | | |
| Clone79 | | | | | | | U | | | | C | | | | | | | | | | | | | |
| Clone62 | | | | | C | | U | | | | C | | | | | | | | | | | | | |
| Clone24 | | | | | | | | | | | C | | | | | | | | | | | | | |
| Clone77 | | | | | | A | | | | | | | | | | | | | | | | | | |
| Clone86 | | | | | | | | | | | | | | | | | | | | | | | | |
| Clone51 | | | | | | | | | | | C | | | | | | | | | | | | | |
| Clone73 | | | | | | | | | C | | | | | | | | | | | | | | | |
| Clone78 | | | | C | | | | | | | C | | | | | | | | | | | | | |
| Clone45 | | U | | | | A | | | | | C | | | | | | | | | | | | | |
| Clone67 | | | | | | | | | | | | | | | | | | | | | | | | |
| Clone65 | | U | | | | A | | | | | C | | | | | | | | | | | | | |
| Clone63 | | | | | | | U | | | | | | | | | | | | | | | | | |
| Clone50 | | | | | | | U | | | | C | | | | | | | | | | | | | |
| Clone39 | | | | | | | | | A | | | | | | | | | | | | | | | |
| Clone50 | | | | | | | U | | | | C | | | | | | | | | | | | | |
| Clone64 | | | | | | | | | | | C | | | | | | | | | | | | | |
| Clone22 | | | | | | | | | | | | | | | | | | | | | | | | |
| Clone23 | | | | | | | U | | A | | C | C | | | | | | | | | | | | |

| Position[a] | 90 | 91 | 92 | 93 | 94 | 95 | 96 | 97 | 98 | 99 | 100 | 101 | 102 | 103 | 104 | 105 | 106 | 107 | 108 | 109 | 110 | 111 | 112 | 113 |
|---|---|---|---|---|---|---|---|---|---|---|---|---|---|---|---|---|---|---|---|---|---|---|---|---|
| Region[b] | | | | P2 | | | | | | | J1-2 | | | | | | P1 | | | | | | | |
| SpiSel | G | U | G | A | G | C | U | C | C | G | U | A | A | C | U | A | G | U | C | G | C | G | U | C |
| Clone68 | | | | G | | | | | | | | | | | | | | | | | | | | |
| Clone34 | | | | G | | | | | | A | | U | G | U | | | | | | | | | | |
| Clone49 | | | | | | | | | | A | | | | | | | | | | | | | | |
| Clone56 | | | | | | | | | | | | | | | | | | | | | | | | |
| Clone46 | | | | | | | | | | A | | | | | | | | | | | | | | A |
| Clone70 | | | | | | | | | | | | G | | | | | | | | | U | | | |
| Clone41 | | | | | | | | | | | | | | U | | | | | | | | | | |
| Clone84 | | | | | | | | | | | | | G | U | | | U | | | | | | | |
| Clone79 | | | | | | | | | | | | | | | | | | | | | | | | |
| Clone62 | | | | | | | G | | | | | | | | | | | | | | U | | | |
| Clone24 | | | | | | | | | | A | | | | x | | | | | U | | U | | | |
| Clone77 | | | | | | | | | | A | | G | | | | G | | | U | | | | | |
| Clone86 | | | | | | | | | | | | G | | | | | | C | | | | | | |
| Clone51 | | | | | | | | | | | | | | | | | | | U | | U | | | |
| Clone73 | | | | | | | | | | A | | | | | x | | | | | | | | | U |
| Clone78 | | | | | | | | | | A | | | G | | | | | | | | | | | |
| Clone45 | | | | | | | | | | | | | | | | | | | | | | | | |
| Clone67 | | | | | | | | | | | | | | | | | | | | | | | | |
| Clone65 | | | | | | | | | | A | | | | | | G | | | | | | | | |
| Clone63 | | | | | | | | | | | x | | | | C | | | | | | G | | | |
| Clone50 | | | | | | | | | U | A | | | | | | | | | | | | | | |
| Clone39 | | | | | | | | | | | | | | | | | | | | A | | | | |
| Clone50 | | | | | | | | | U | A | | | | | | | | | | | | | | |
| Clone64 | | | | | | | | | | | C | | | | | | | | U | | | | | U |
| Clone22 | | | | | | | | | | A | | | | | | | | | | | U | | | |
| Clone23 | | | | | | | | | | | | | | | | | | | A | | | | | |

Table 3- part 2 represents the sequences of isolated mutants. Black crosses represent deletions.

[a] Positions numbered according to SpiSel sequence

[b] Regions labeled according to the model proposed in (2)

## Miniaturization and engineering of the improved Spinach

Prior to individually evaluating the contribution of each mutation, the inventors defined the minimal size of 4-68. Indeed, in a previous study Spinach was successfully shortened down to a 51 nucleotides long molecule (BabySpinach) that retained fluorogenic properties (21).

Sequence alignment of the most fluorescent variants revealed that a significant variability (especially deletions) was found in J1/2 (**Table 3**) region indicating a dispensable role of this domain. Accordingly, 4-68-J1/2, a variant deleted of J1/2 (**Figure 3A**), conserved intact fluorogenic properties (**Figure 3B**). Conversely, the G-quadruplex domain was strongly conserved and, within the sampled population, only one mutation (A42U) was tolerated (mutant 49 in **Table 3**). Transplanting this mutation into 4-68-J1/2 afforded the variant 4-68/49 (**Figure 3A**) that conserved identical fluorogenic properties in potassium-containing buffer but had a fluorescence increased ~3 times in sodium-containing buffer (**Figure 3B**). This improvement was likely to result from a higher stabilization of the G-quadruplexes in sodium and at 37°C when U was present at this position. Interestingly, a recent thermodynamic-based scanning of Spinach, also identified this mutation as the only one tolerated in this region of the molecule, further supporting these results (25).

The aptamer miniaturization process was pursued by removing 6 base pairs from P3 region to generate a shorter and continuous apical helix (mutant min68). In addition, the first 3 or 6 base pairs of P1 were deleted to generate respectively variants min68/49-3 and min68/49-6 (**Figure 3A**). While the fluorescence of both constructs remained identical to that of 4-68/49 in the absence of constant regions (**Figure 3C**), their presence drastically reduced the fluorescence of min68/49-6. Conversely, the fluorescence of the longer variant min68/49-3 was unaffected by the constant regions. Even though more experiments are necessary, it is tempting to propose that the long P1/2 helix of min68/49-3 could act as an insulation module, similar to a three-way junction recently engineered (33), that protects the aptamer from the surrounding sequences.

In addition to A17U (originally A42U), the inventors' miniaturized variant (min68/49-3) still contained 6 of the 8 original mutations. U8C and U39C were likely to act by stabilizing RNA structure by converting G.U in stronger G-C base pairs. Mutations A14G and A56G were always found associated together and were the only common feature shared by the two most active mutants (clone 68 and 34 in **Table 3**). To test the contribution of this pair of mutations, the inventors produced a double mutant G14A/G56A and measured its capacity to form a fluorescent complex with DFHBI. Whereas reverting these mutations had only a small effect in the presence of potassium, they drastically reduced the fluorescence in other salts pointing a key role of these mutations in salt resistance of the molecule (**Figure 3D**). Their location may suggest that these mutations could form a third G-quadruplex with surrounding Gs and, doing so, increase the overall stability of the aptamer. To shed light on the contribution of the two last mutations (C31 U and U32C) found in the apical loop of the aptamer, the inventors generated a second double mutant (U31C/C32U) and tested its capacity to form fluorescent complex with DFHBI. Surprisingly, reverting these two positions was found to increase the fluorescence of the complex by ~25% whereas its salt resistance stayed unchanged (**Figure 3D**). The inventors considered this double mutant, (U31C/C32U) of min68/49-3, to be the optimal form of the aptamer that could be isolated by the inventors' approach and they named it iSpinach.

## Performances and applications of iSpinach

The inventors next compared the performances of iSpinach and Spinach2 (**Figure 4**). The inventors found that, in addition to being 1.3 times smaller then Spinach2, iSpinach was 1.4 times more fluorescent in potassium (**Figure 1**). This improvement was likely a combined effect of slightly better folding ability of iSpinach (F.E. ~70%), a twice-higher affinity for the DFHBI and a slightly increased molar fluorescence of the DFHBI in complex with iSpinach (**Figure 4**). The inventors noticed that $K_d$ values obtained with Spinach2 were slightly higher than those from the literature. The inventors attributed this difference to the temperature (25°C) used in these measurements. Indeed, the inventors found that $K_d$ values increased with the temperature (**Figures 8**). However, since none of the previous studies indicated the temperature used to measure this constant, more accurate comparison could not be performed. Finally, iSpinach also formed a more thermostable complex with DFHBI displaying a melting temperature $(T_m^{cplx})$ increased by more than 8°C (**Figure 4**). iSpinach also outperformed Spinach2 in the presence of lithium and cesium, but the inventors were more surprised to find that the iSpinach/DFHBI complex was not only stable in the presence of sodium but even twice more fluorescent than with potassium (**Figure 1**). This impressive improvement of the molecule resulted from a better capacity of the aptamer to fold (F.E. ~ 60%) as compared with Spinach2 leading to an affinity for DFHBI increased by a least an order of magnitude, as well as a doubling of the molar fluorescence of DFHBI (**Figure 4**). At this stage, only speculations can be proposed to explain how the nature of the cation affects the brightness of the complex.

The improved folding and fluorescence properties of iSpinach in a wider range of conditions should make it appealing for the design of novel fluorogenic probes as well as of sensitive fluorogenic assays. To demonstrate the advantages that iSpinach may offer, the inventors set-up an *in vitro* experiment aiming at measuring the apparent catalytic constant $(k_{cat}^{app})$ of a ribozyme directly from the *in vitro* transcription mixture. As model ribozyme, the inventors choose iXm1, a variant of X-motif the inventors recently isolated and which transforms its substrate though a simple first order kinetics (29). The inventors fused the DNA coding either for iSpinach, or for Spinach2, to the 3' end of iXm1 coding region and placed the construct under the control of T7 RNA polymerase promoter sequence (**Figure 5A**). The resulting gene was then *in vitro* transcribed in a mixture containing a low concentration of T7 RNA polymerase (50 times less than for expression in droplets) to limit the amount of ribozyme generated in the reaction mixture and properly assess $k_{cat}^{app}$ values. In addition, since X-motif was shown not to require monovalent cations (34), the reaction mixture was not supplemented with extra monovalent cations. Upon DNA addition, the reaction mixture was immediately split and mixed with either DFHBI or S21-Atto (a fluorogenic substrate of iXm1(29)) and the fluorescence monitored at 37°C. Ribozyme activity was readily measurable and was not affected by the fusion of the catalyst with fluorogenic aptamers (**Figure 5B**). Conversely, DFHBI fluorescence was observed only when iSpinach was used alone or fused with iXm1, whereas no fluorescence was observed in the presence of Spinach2 (**Figure 5C**). The lack of fluorescence of Spinach2-based constructs was attributed to the poor folding performance of the Spinach2 in the experimental conditions used (i.e. potassium-free reaction mixture incubated at 37°C). Spinach2 folding inefficiency could be partly overcome by increasing the potassium concentration up to 100 mM (Figure 1) (20), a concentration of monovalent cations also shown to strongly inhibit T7 RNA polymerase activity (35). Therefore, in addition of being brighter, iSpinach allows to perform experiments in conditions optimal for the activity of the polymerase. The co-transcriptional kinetics of iXm1 activity could be approximated by equation (1):

$$\frac{\mathrm{dP}}{\mathrm{dt}} = k_{cat}^{app}\left(\sigma^{\mathrm{rbz}} - \delta^{\mathrm{rbz}}\right) + k_{uncat} \qquad (1)$$

with $\sigma^{rbz}$ the synthesis rate of the ribozyme, $\delta^{rbz}$ its degradation rate and $k_{uncat}$ the substrate auto-hydrolysis rate resulting form T7 RNA polymerase side activity (29). Considering the high efficiency of T7 RNA polymerase, $\delta^{\mathrm{rbz}}$ was neglected, whereas $\sigma^{rbz}$ and $k_{uncat}$ were experimentally measured to be 2.1 pmol/min and 0,17 pmol/min respectively and allowed extracting a $k_{cat}^{app}$ value of 0.36 $min^{-1}$, a value that is close to the 0.48 $min^{-1}$ previously measured with gel-purified RNA (29). This fluorogenic approach can be used to screen ribozyme mutant libraries in high throughput regimes for identifying molecules with high $k_{cat}^{app}$, but it also demonstrate how the performances of iSpinach is advantageous. In addition, changing the dye labeling the fluorogenic substrate of X-motif for a red emitting one would allow for simultaneously monitoring RNA synthesis (*via* iSpinach fluorescence) and ribozyme activity in a single well.

The present work allowed the inventors to develop iSpinach, a DFHBI-binding aptamer with properties (thermal stability and brightness) surpassing those of Spinach2 and all other DFHBI-binding aptamers in the conditions tested. Isolating this new optimized aptamer was possible by using selection pressures difficult, if not impossible, to apply on living cells (i.e. high temperature, potassium-free environment) but easy to implement *in vitro* using μIVC. In addition, evolving fluorogenic aptamers *in vivo* faces cell-size variations issue making necessary the use of a second fluorescent reporter as internal reference (4), whereas the strong monodispersity (polydispersity < 3% (29)) and homogeneity of the droplets used in μIVC makes this dispensable.

iSpinach acquired several mutations that might have been difficult to predict, especially in the absence of a crystal structure model. The work of the inventors shows how powerful *in vitro* evolution may be, provided proper selection pressures and selection format are used. Indeed, whereas Spinach2 was obtained by rational design, the tRNA scaffold-free molecule remained sub-optimal in term of fluorescence and folding stability. In addition, one should also note that none of Spinach2 mutations was found in iSpinach. Recently, an alternative thermodynamic-based approach using large-scale integrated microfluidic chips was introduced to screen libraries of point mutants of Spinach (25). Whereas this technology is extremely powerful at screening small libraries of mutants carrying single mutations, its limited throughput (<1000 mutants per round of screening) makes it less suited at screening more complex libraries (≥2 mutations per molecule). Consequently, even though part of iSpinach mutations were revealed by this methodology, none of them was considered and the improved version of the aptamer was both less thermostable and less fluorescent than iSpinach. The improved properties of iSpinach make it appealing for the development of new *in vitro* applications such as biosensing or high throughput screening. The wider salt-tolerance of iSpinach also makes it a good candidate for the development of new fluorogenic probes working in cellular ionic microenvironments (e.g. endosomes and organelles) to sense their composition (e.g. metabolites, proteins) as this was already described with DNA-based nanodevices (36).

The main limitation of iSpinach and of every other DFHBI-binding aptamer is their low affinity for the DFHBI

($K_d$ > 400 nM) compared to what could be expected. However, Mango RNA was recently isolated and shown to have an affinity 2 order of magnitude higher for its fluorogenic co-factor (*Kd* ~3nM) (10). One can therefore predict that performing SELEX in more stringent selection conditions that favor the isolation of high affinity binders in tandem with μIVC screening should make possible to isolate aptamers able to form bright and high affinity complexes. Finally, Spinach/DFHBI complex was also shown to be prone to light-induced dissociation (37). Since the inventors' screening is based on light-induced fluorescence measurement, the inventors anticipate that, in the future, using a stronger light source as well as increasing the illumination time may offer the possibility to isolate new mutants with improved photostability.

**Example 2 - iSpinach properties in extracellular environment**

In order to estimate how good iSpinach could be for a use in extracellular environment, the inventors recorded its fluorescence and compared it to that of Spinach2 in HL3 (5 mM Hepes, 10 mM $NaHCO_3$, 110 mM NaCl, 5mM KCl, 10 mM $MgCl_2$, 30 mM Sucrose, 5 mM Threalose), a medium mimicking the hemolymph and used to grow insect cells.
Results are shown in **Figure 10**.
As previously shown in Example 1, a much higher fluorescence was obtained with iSpinach compared to Spinach 2 whatever the temperature used. These data confirm the iSpinach superior properties in the extracellular environment compared to another aptamer.

**Example 3 - Use of iSpinach for intracellular imaging**

The inventors also verified that iSpinach could be properly expressed *in vivo.* To do so, iSpinach was inserted into a tRNA-scaffold and the gene introduced into a plasmid (pET-28) under the control of T7 RNA polymerase. E.coli bacteria (BL21 DE3) were transform with the construct and grown in the presence of 2% glucose until entering in exponential growth phase. Bacteria were then pelleted and resuspended in LB medium supplemented with 1 mM IPTG and incubated for 3h00 to induce T7 RNA polymerase production and aptamer synthesis. Bacteria were then washed and incubated for 30 minutes at 37°C in PBS supplemented with 20 mM glucose, 1 mM $CaCl_2$, 5 mM $MgSO_4$ and 200μM DFHBI-1T. Bacteria were finally washed in PBS and analyzed on a fluorescence microscope.
Results are shown in **Figures 11A to 11D**.
Whereas no significant fluorescence can be observed on the negative control (bacteria transformed with plasmid containing a gene coding for a non-DFHBI-binding aptamer), and that bacteria were easily observed in bright field, a strong green fluorescence was observed in the presence of iSpinach, confirming that iSpinach may also be used for *in vivo* gene expression imaging.

**Bibliographic references**

1. Arora, A., et al. (2015) Nucleic acids research.
2. Babendure, J.R., et al. (2003) J Am Chem Soc, 125, 14716-14717.
3. Constantin, T.P., et al. (2008) Organic letters, 10, 1561-1564.
4. Filonov, G.S., (2014) J Am Chem Soc, 136, 16299-16308.
5. Lee, J., (2010) ACS chemical biology, 5, 1065-1074.
6. Paige, J.S., et al. (2011) Science, 333, 642-646.
7. Pei, R., et al. (2009) Nucleic acids research, 37, e59.
8. Sando, S., (2008) Chemical communications, 3858-3860.
9. Sparano, B.A. and Koide, K. (2005) J Am Chem Soc, 127, 14954-14955.
10. Dolgosheina, E.V., et al. (2014) ACS chemical biology.
11. Strack, R.L. and Jaffrey, S.R. (2015) Methods in enzymology, 550, 129-146.
12. Sunbul, M. and Jaschke, A. (2013) Angewandte Chemie, 52, 13401-13404.
13. Sato, S., et al. (2015) Angewandte Chemie, 54, 1855-1858.
14. Paige, J.S., et al. (2012) Science, 335, 1194.
15. You, M., et al. (2015) Proc Natl Acad Sci USA, 112, E2756-2765.
16. Hofer, K., et al. (2013) J Am Chem Soc, 135, 13692-13694.
17. Chudakov, et al. (2010) Physiological reviews, 90, 1103-1163.
18. You, M. and Jaffrey, S.R. (2015)Annual review of biophysics, 44, 187-206.
19. Strack, R.L., et al. (2013) Nature methods, 10, 1219-1224.
20. Huang, H., et al. (2014) Nature chemical biology, 10, 686-691.

21. Warner, K.D., et al. (2014) Nature structural & molecular biology, 21, 658-663.
22. Ellington, A.D. and Szostak, J.W. (1990) Nature, 346, 818-822.
23. Stoltenburg, R., et al. (2007) Biomolecular engineering, 24, 381-403.
24. Tuerk, C. and Gold, L. (1990) Science, 249, 505-510.
25. Ketterer, S., et al. (2015) Nucleic acids research.
26. Bhadra, S. and Ellington, A.D. (2014) RNA, 20, 1183-1194.
27. Rogers, T.A., et al. (2015) ACS synthetic biology, 4, 162-166.
28. Sharma, S., et al.. (2014) Small, 10, 4276-4280.
29. Ryckelynck, M., et al. (2015) RNA, 21, 458-469.
30. Ponchon, L. and Dardel, F. (2007) Nature methods, 4, 571-576.
31. Shabala, L., et al. (2009) Environmental microbiology, 11, 137-148.
32. Pedelacq, J.D., et al. (2006) Nat Biotechnol, 24, 79-88.
33. Filonov, G.S., et al. (2015) Chemistry & biology, 22, 649-660.
34. Lazarev, D., et al. (2003) RNA, 9, 688-697.
35. Chamberlin, M. and Ring, J. (1973) The Journal of biological chemistry, 248, 2245-2250.
36. Saha, S., et al. (2015) Nature nanotechnology, 10, 645-651.
37. Han, K.Y, et al. (2013) J Am Chem Soc, 135, 19033-19038.

## Claims

1. A nucleic acid molecule capable of binding to a fluorophore molecule, the nucleic acid molecule being a molecule containing a first and a second region, said first and second regions being such that:

   - the first region comprises the nucleotide sequence of SEQ ID NO: 1 ; and
   - the second region comprises the nucleotide sequence of SEQ ID NO: 2 ,

   preferably, wherein the first and the second regions present at least a partial base pairing.

2. The nucleic acid molecule according to claim 1, said nucleic acid molecule being a linear single-stranded molecule, a circular single-stranded molecule or a two-stranded molecule.

3. The nucleic acid molecule according to claim 1 or 2, wherein the nucleic acid molecule comprises one of the nucleotide sequence of

   - $(N)_a$UGAGGGUCGGGUCC$(N)_b(N)_c(N)_b$GUUGAGUAGAGUGUGGGCU$(N)_a$ (SEQ ID NO: 3),
   - $(N)_a$GUUGAGUAGAGUGUGGGCU$(N)_b(N)_c(N)_b$UGAGGGUCGGGUCC$(N)_a$ (SEQ ID NO: 4), or

   the two following sequences

   - $(N)_a$UGAGGGUCGGGUCC$(N)_b(N)_d$ (SEQ ID NO: 5), $(N)_a$GUUGAGUAGAGUGUGGGCU$(N)_b(N)_d$ (SEQ ID NO: 6),

   wherein a, b , c and d are integer

a is higher than or equal to 4, preferably varies from 4 to 100,
b is higher than or equal to 5, preferably varies from 5 to 50,
c is higher than or equal to 4, preferably varies from 4 to 200,

and 2d=c.

**4.** The nucleic acid molecule according to claim 1 or 2, wherein the nucleic acid molecule comprises one of the nucleotide sequence of

- SEQ ID NO: 7 or SEQ ID NO: 8, or
- both sequences of SEQ ID NO: 9 and SEQ ID NO: 10.

**5.** The nucleic acid molecule according to anyone of claims 1 to 4, wherein the nucleic acid molecule consists or consists essentially of the nucleotide sequence of SEQ ID NO: 11.

**6.** The nucleic acid molecule according to claim 1 , wherein the fluorophore molecule is a fluorophore comprising a methyne bridge between a substituted aromatic ring system and a substituted imidazol(thi)one, oxazol(thi)one, pyrrolin(thi)one, or furan(thi)one.

**7.** A molecular complex comprising:

- a fluorophore molecule comprising a methyne bridge between a substituted aromatic ring system and a substituted imidazol(thi)one, oxazol(thi)one, pyrrolin(thi)one, or furan(thi)one, in particular DFHBI ; and
- the nucleotide molecule according to one of claims 1 to 6 specifically bound to the fluorophore molecule.

**8.** A DNA molecule coding for a nucleic acid molecule according to anyone of claims 1 to 6.

**9.** A genetically engineered DNA molecule comprising a first region encoding a nucleic acid molecule according to anyone of claims 1 to 6.

**10.** The genetically engineered DNA molecule according to claim 9 further comprising a promoter operably coupled to the first region to control expression of the RNA molecule.

**11.** A host cell containing the nucleic acid molecule as defined in any one of claims 1 to 6, or a molecular complex according to claim 7, or containing the DNA molecule according to claim 8, or containing the genetically engineered DNA molecule as defined in claim 9 or 10, or a combination thereof.

**12.** A host cell according to claim 11, further containing a fluorophore molecule comprising a methyne bridge between a substituted aromatic ring system and a substituted imidazol(thi)one, oxazol(thi)one, pyrrolin(thi)one, or furan(thi)one, in particular DFHBI.

**13.** A detection array comprising one or more nucleic acid molecules according to one of claims 1 to 6 tethered to a discrete location on a surface of the array.

**14.** Use of
the nucleic acid molecule according to anyone of claims 1 to 6, or
the molecular complex according to claim 7, or
the DNA molecule coding for a nucleic acid molecule according to claim 8, or
the genetically engineered DNA molecule according to claim 9 or 10, or
the host cell according to claim 11 or 12,
or a combination thereof,
for imaging in vitro or ex vivo small molecules, RNA and proteins.

**15.** A method for imaging in vitro or ex vivo small molecules, RNA and proteins in cells, comprising the administration a living in vivo and ex vivo cultures cell a nucleic acid according to anyone of claims 1 to 6, operably linked to a RNA, along with a fluorophore molecule comprising a methyne bridge between a substituted aromatic ring system and a substituted imidazol(thi)one, oxazol(thi)one, pyrrolin(thi)one, or furan(thi)one, in particular DFHBI.

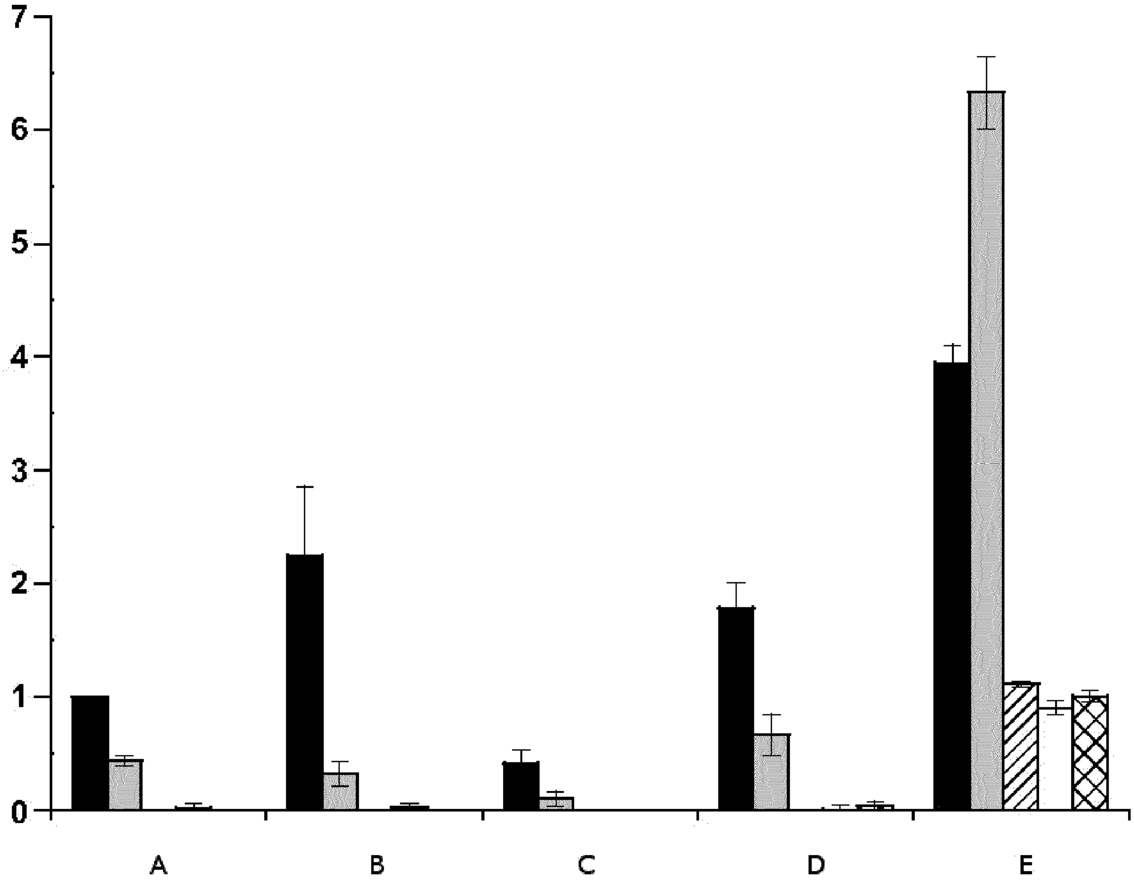


**Figure 1**

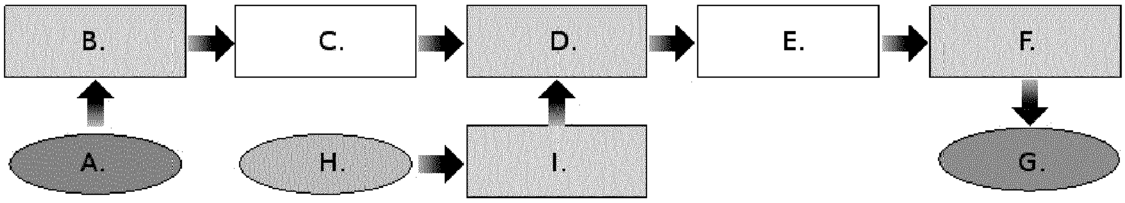


Figure 2A

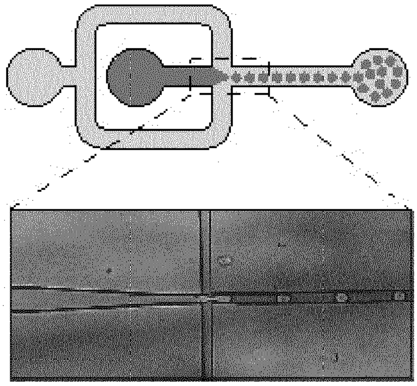

Figure 2B

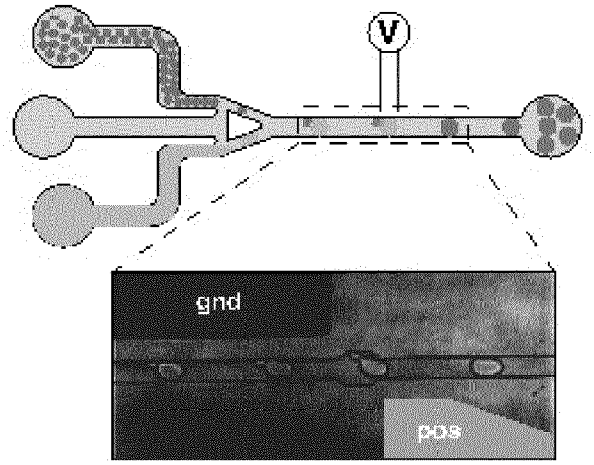


Figure 2C

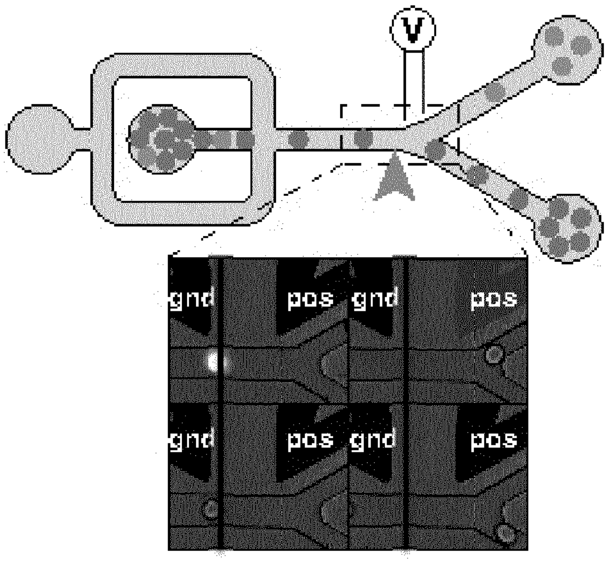


Figure 2D

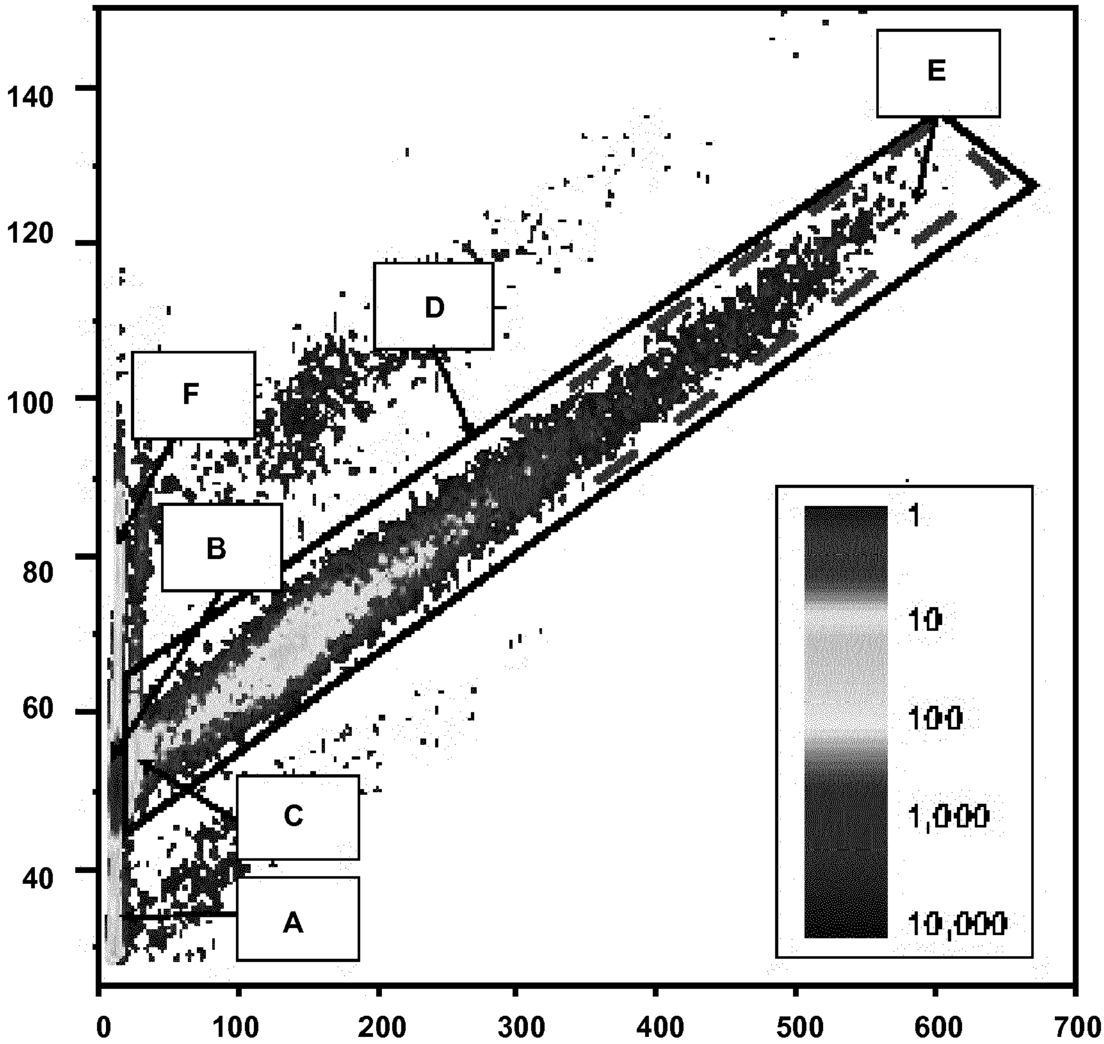
140
120
100
80
60
40
0
100
200
300
400
500
600
700
A
B
C
D
E
F
1
10
100
1,000
10,000

**Figure 2E**

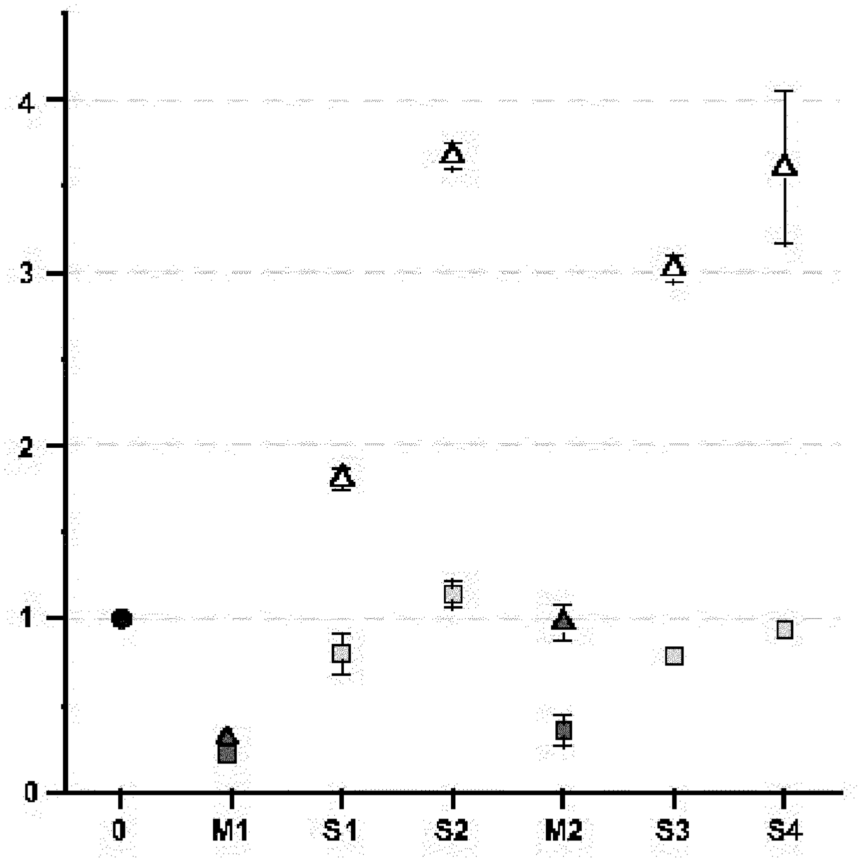


Figure 2F

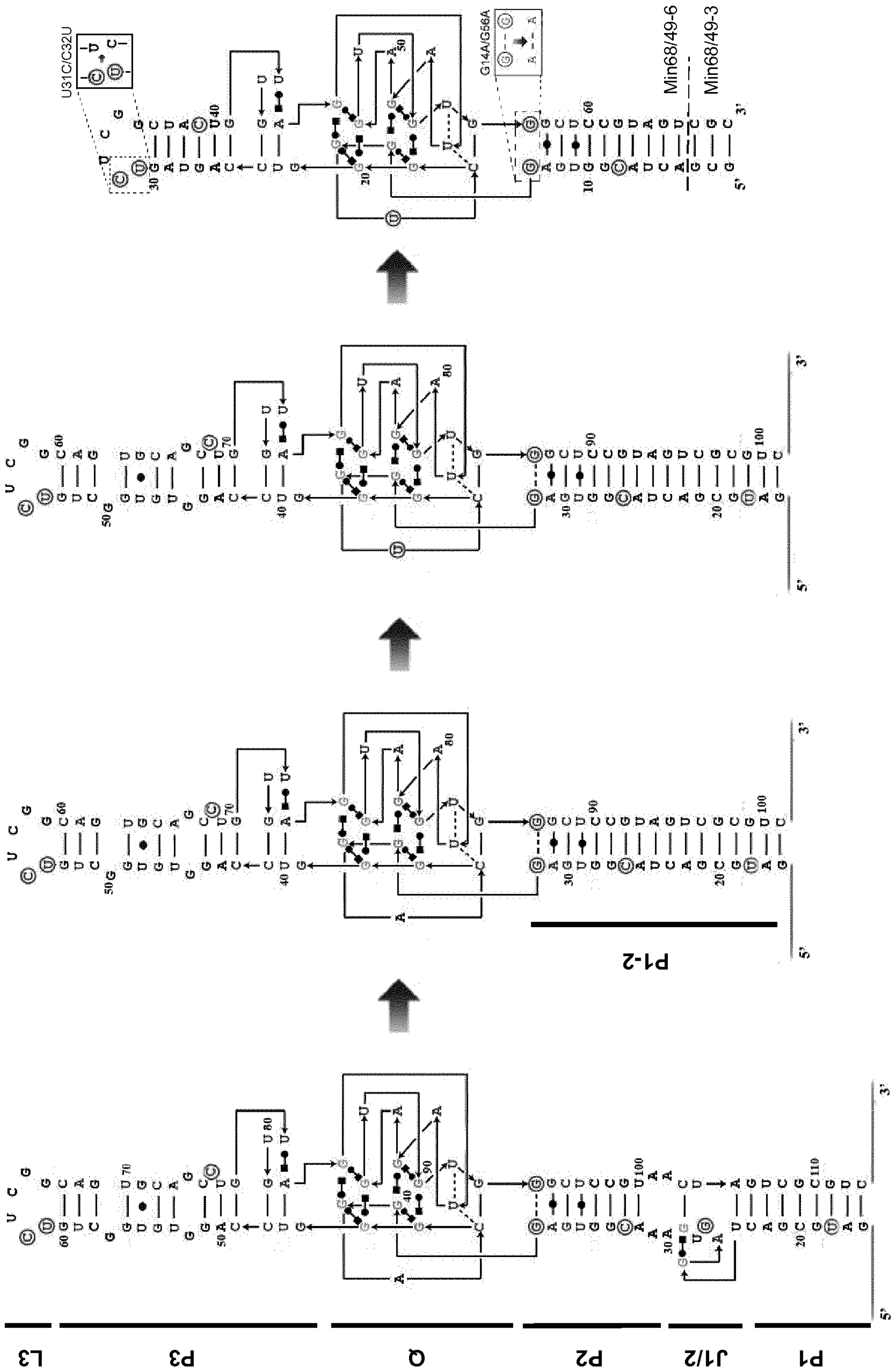


Figure 3A

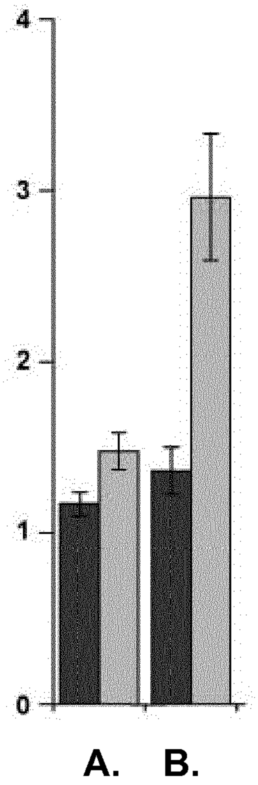


Figure 3B

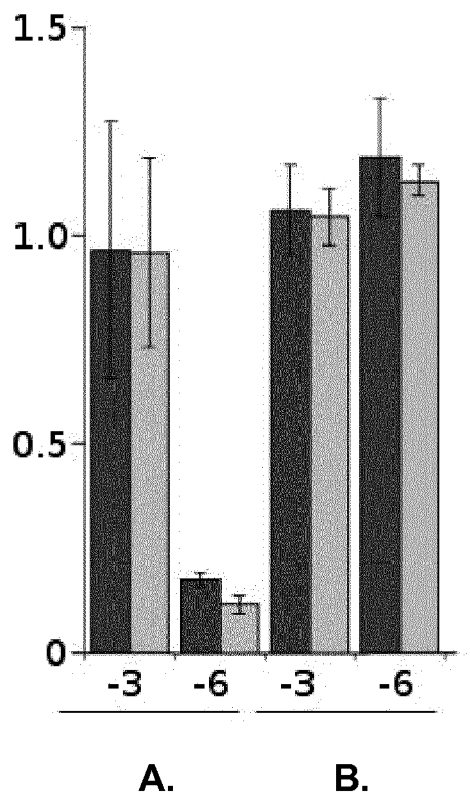


Figure 3C

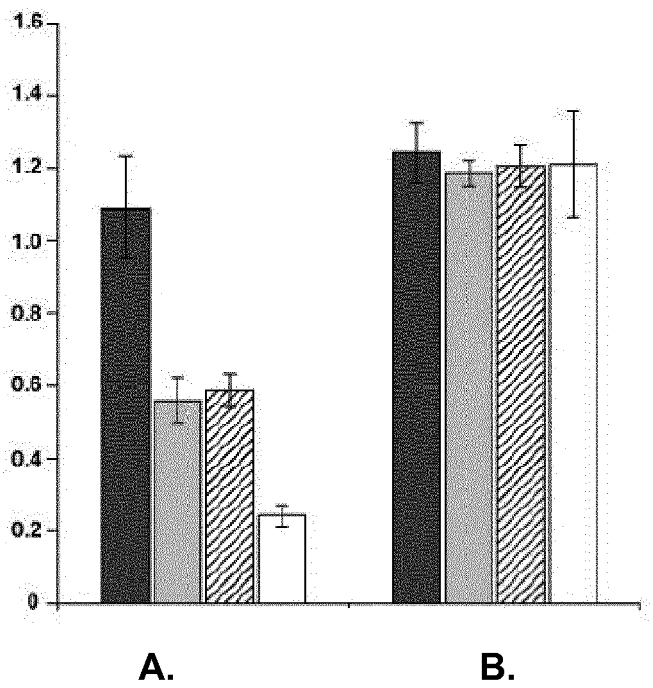


Figure 3D

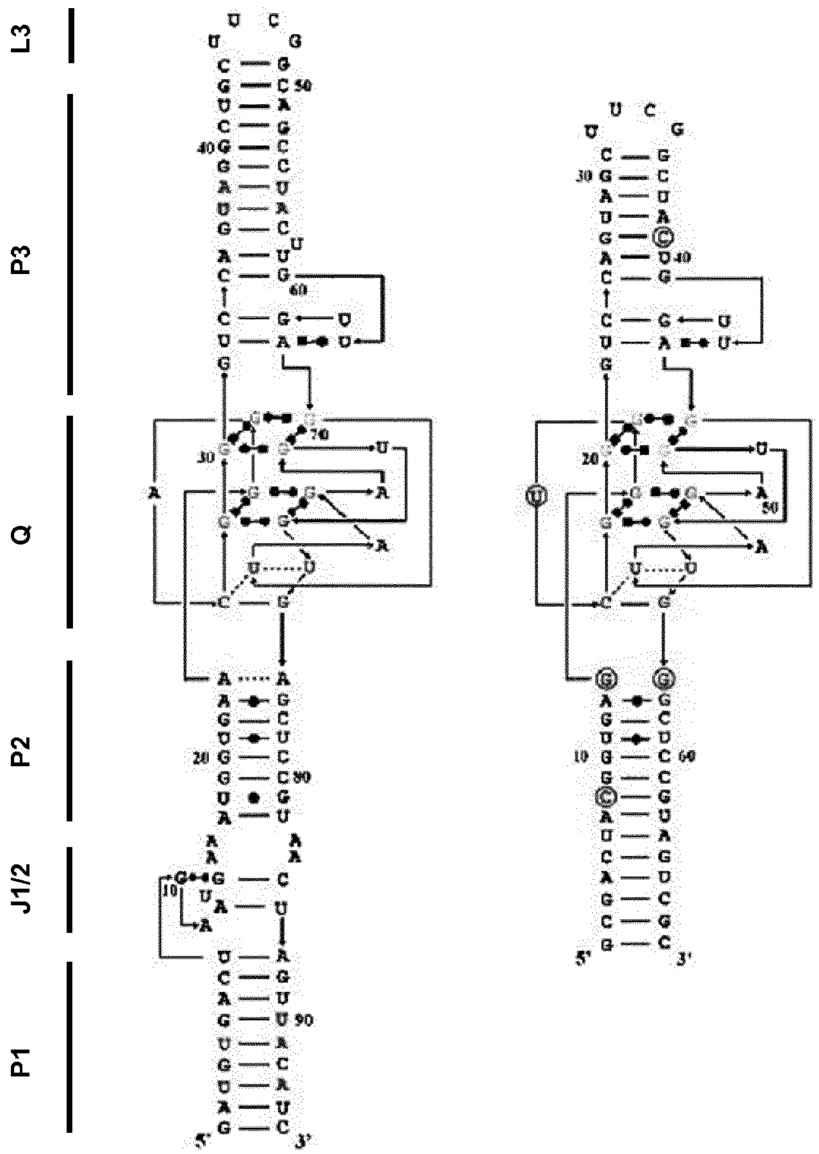


| | Spinach 2.0 | | iSpinach | |
|---|---|---|---|---|
| | K+ | Na+ | K+ | Na+ |
| FE (%) | 61.0 ± 12.2 | n.d | 69.0 ± 4.0 | 59.6 ± 8.1 |
| $T_m^{cplx}$ (°C) | 36.2 ± 0.2 | 30.6 ± 0.3 | 44.6 ± 0.2 | 36.3 ± 0.1 |
| $K_d$ (μM) | 1.45 ± 0.2 | >53.4 ± 7 | 0.92 ± 0.1 | 4.46 ± 0.2 |
| Max ex. (nm) | 445 | n.d | 442 | 458 |
| Max em. (nm) | 501 | n.d | 503 | 507 |
| Rel. Molar Fluo of DFHBI | 1 | n.d | 1.4 ± 0.1 | 2.1 ± 0.2 |

**Figure 4**

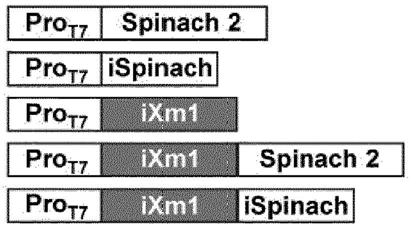


**Figure 5A**

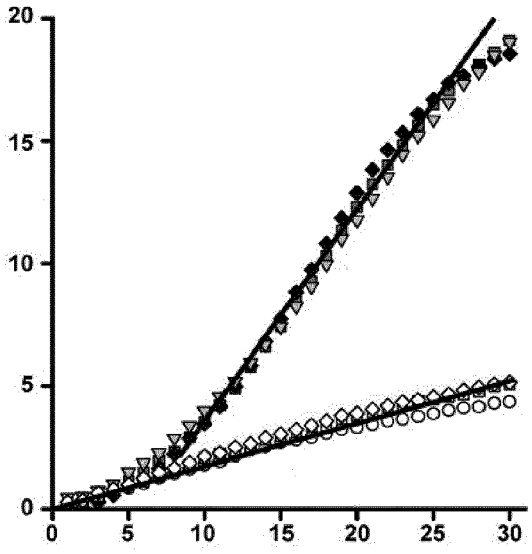


**Figure 5B**

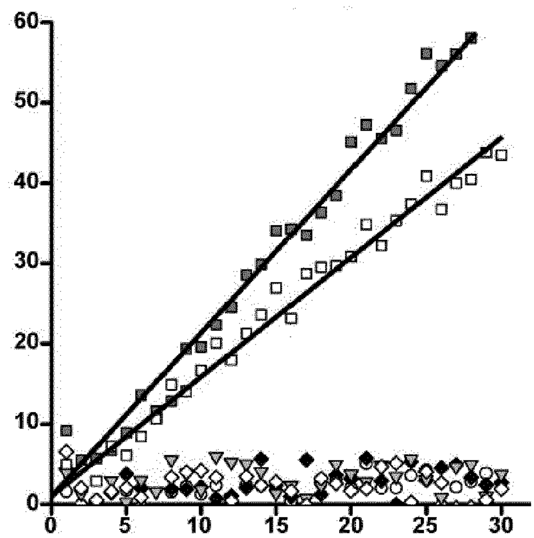


**Figure 5C**

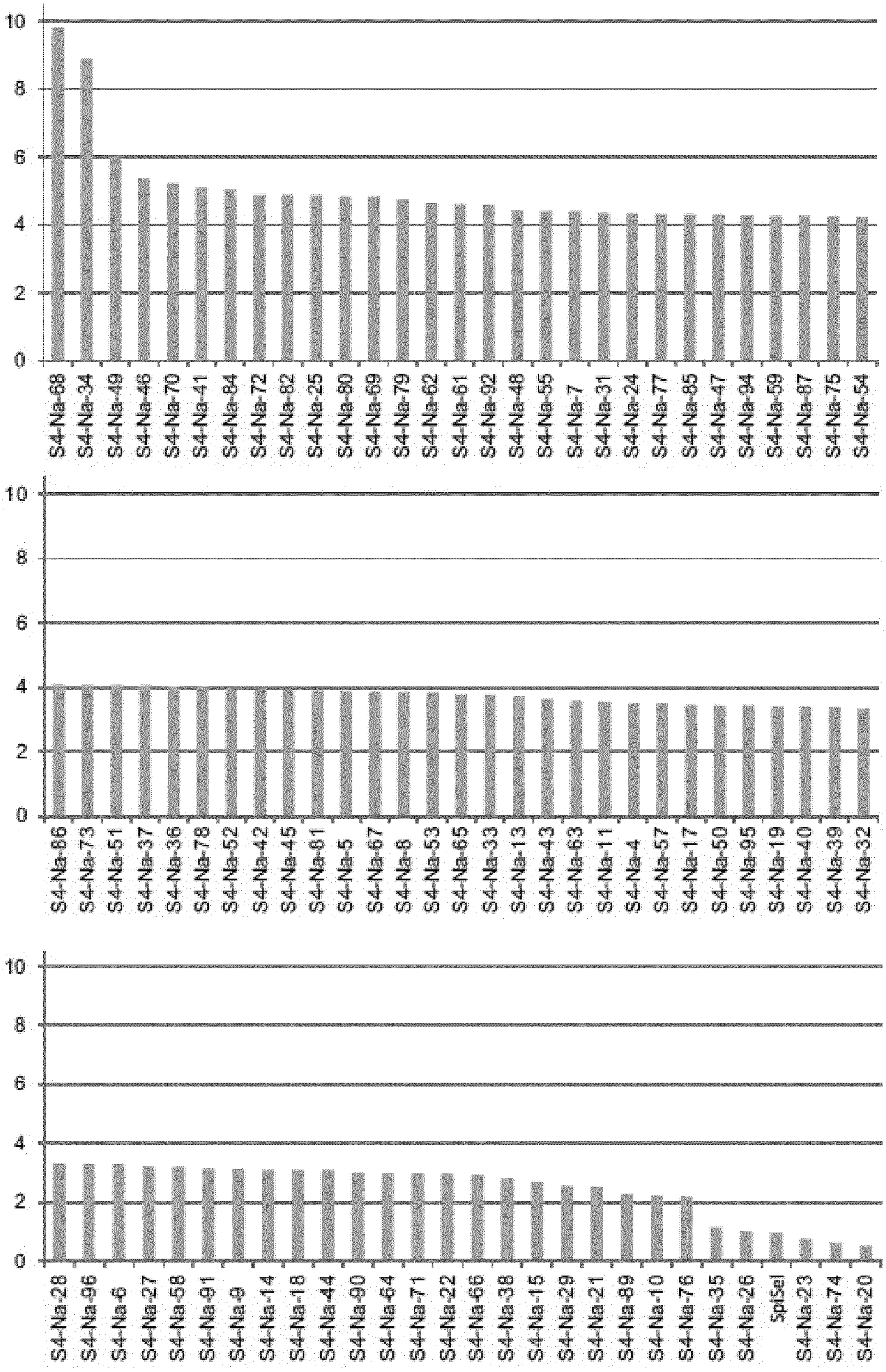
10
8
6
4
2
0
S4-Na-68
S4-Na-34
S4-Na-49
S4-Na-46
S4-Na-70
S4-Na-41
S4-Na-84
S4-Na-72
S4-Na-82
S4-Na-25
S4-Na-80
S4-Na-69
S4-Na-79
S4-Na-62
S4-Na-61
S4-Na-92
S4-Na-48
S4-Na-55
S4-Na-7
S4-Na-31
S4-Na-24
S4-Na-77
S4-Na-85
S4-Na-47
S4-Na-94
S4-Na-59
S4-Na-87
S4-Na-75
S4-Na-54
10
8
6
4
2
0
S4-Na-86
S4-Na-73
S4-Na-51
S4-Na-37
S4-Na-36
S4-Na-78
S4-Na-52
S4-Na-42
S4-Na-45
S4-Na-81
S4-Na-5
S4-Na-67
S4-Na-8
S4-Na-53
S4-Na-65
S4-Na-33
S4-Na-13
S4-Na-43
S4-Na-63
S4-Na-11
S4-Na-4
S4-Na-57
S4-Na-17
S4-Na-50
S4-Na-95
S4-Na-19
S4-Na-40
S4-Na-39
S4-Na-32
10
8
6
4
2
0
S4-Na-28
S4-Na-96
S4-Na-6
S4-Na-27
S4-Na-58
S4-Na-91
S4-Na-9
S4-Na-14
S4-Na-18
S4-Na-44
S4-Na-90
S4-Na-64
S4-Na-71
S4-Na-22
S4-Na-66
S4-Na-38
S4-Na-15
S4-Na-29
S4-Na-21
S4-Na-89
S4-Na-10
S4-Na-76
S4-Na-35
S4-Na-26
SpiSel
S4-Na-23
S4-Na-74
S4-Na-20

**Figure 6**

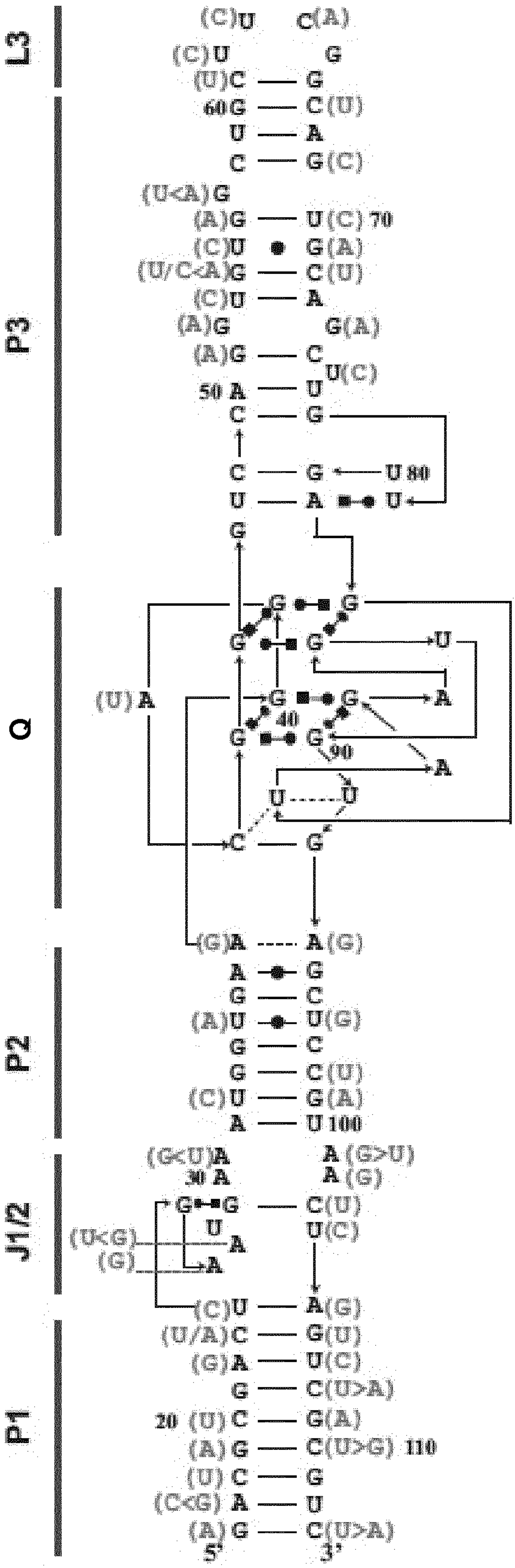


Figure 7A

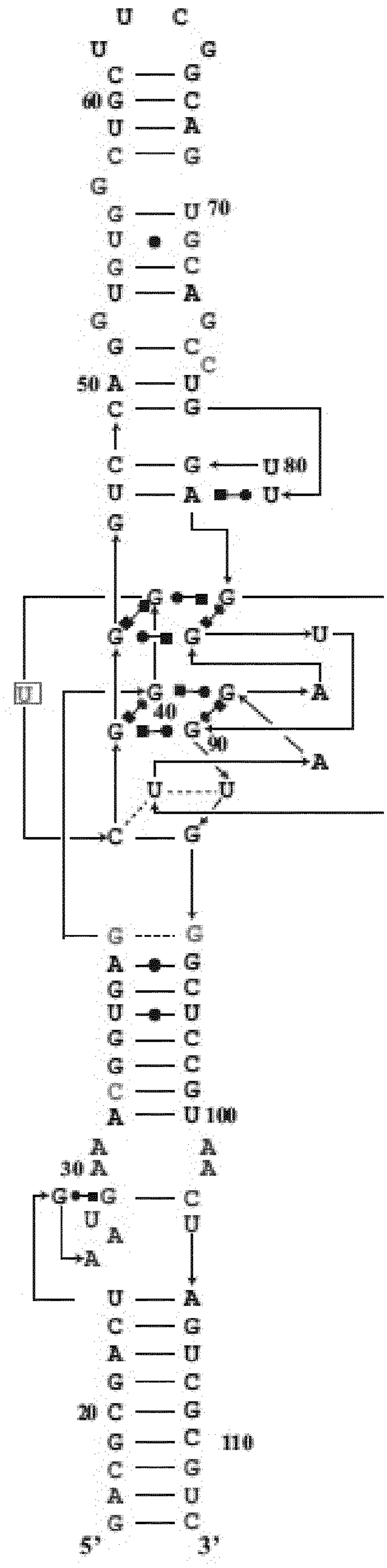


Figure 7B

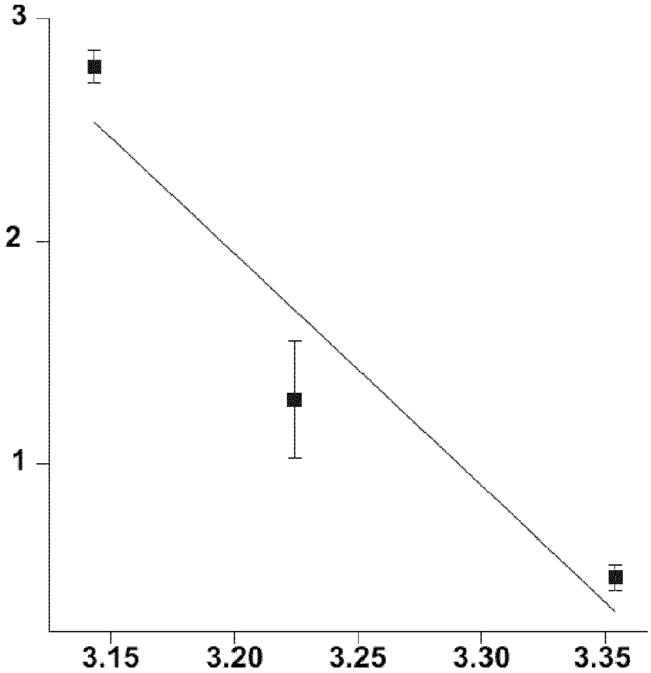


Figure 8A

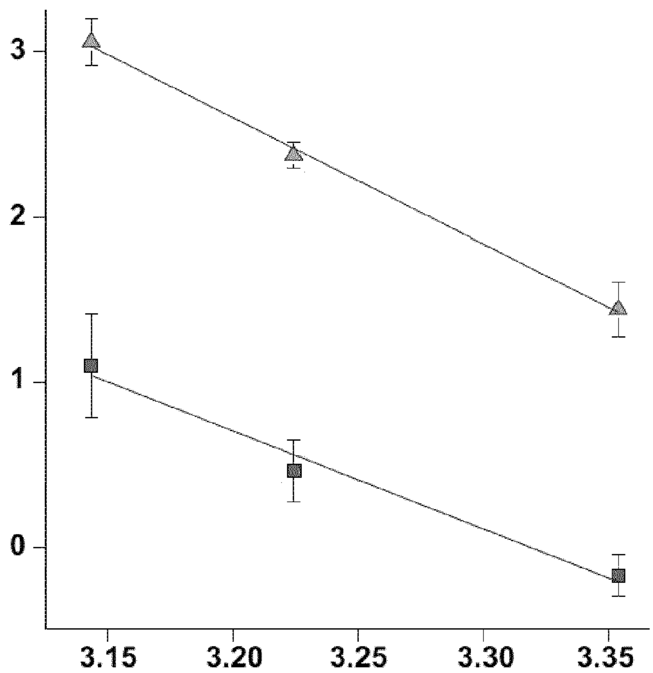


Figure 8B

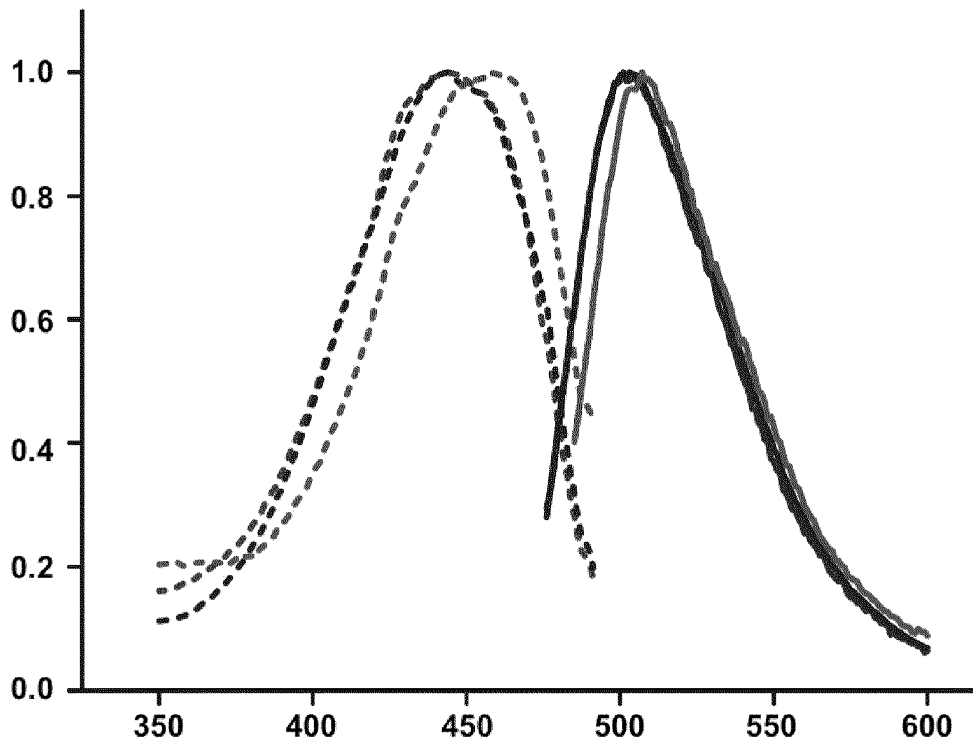


Figure 9

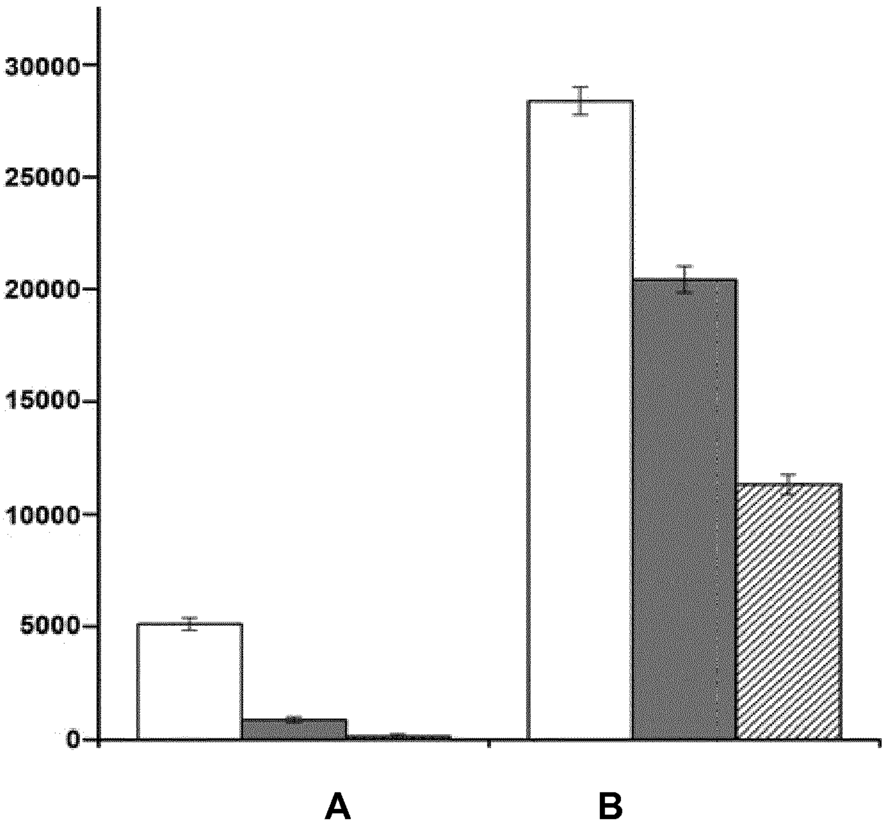
30000
25000
20000
15000
10000
5000
0
A
B

Figure 10

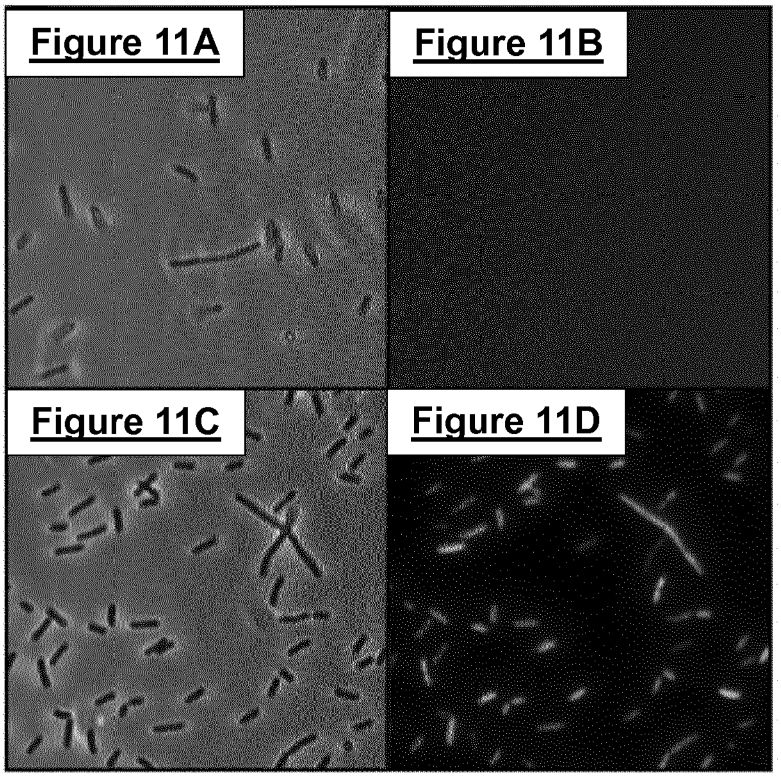
Figure 11A
Figure 11B
Figure 11C
Figure 11D

Europäisches Patentamt
European Patent Office
Office européen des brevets

# EUROPEAN SEARCH REPORT

Application Number
EP 16 30 5224

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SIMON KETTERER ET AL: "Systematic reconstruction of binding and stability landscapes of the fluorogenic aptamer spinach", NUCLEIC ACIDS RESEARCH, vol. 43, no. 19, 30 October 2015 (2015-10-30), pages 9564-9572, XP055292322, GB ISSN: 0305-1048, DOI: 10.1093/nar/gkv944 * figure s8 * ----- | 1-15 | INV. C12N15/115 |
| A | WO 2013/016694 A2 (UNIV CORNELL [US]; JAFFREY SAMIE R [US]; PAIGE JEREMY [US]) 31 January 2013 (2013-01-31) * figures 3a,b * ----- | 1-15 | |
| A | US 2015/141282 A1 (JAFFREY SAMIE R [US] ET AL) 21 May 2015 (2015-05-21) * figures 1,18,19 * ----- | 1-15 | TECHNICAL FIELDS SEARCHED (IPC) |
| T | ALEXIS AUTOUR ET AL: "iSpinach: a fluorogenic RNA aptamer optimized for in vitro applications", NUCLEIC ACIDS RESEARCH, vol. 44, no. 6, 7 April 2016 (2016-04-07), pages 2491-2500, XP055292326, GB ISSN: 0305-1048, DOI: 10.1093/nar/gkw083 ----- | | C12N |

The present search report has been drawn up for all claims

1

| Place of search | Date of completion of the search | Examiner |
|---|---|---|
| The Hague | 29 July 2016 | Romano, Alper |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 30 5224

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2016

| Patent document cited in search report | Publication date | Patent family member(s) | Publication date |
|---|---|---|---|
| WO 2013016694 A2 | 31-01-2013 | US 2014220560 A1<br>WO 2013016694 A2 | 07-08-2014<br>31-01-2013 |
| US 2015141282 A1 | 21-05-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010096584 A
- WO 2013016694 A

### Non-patent literature cited in the description


- **ARORA, A. et al.** *Nucleic acids research,* 2015
- **BABENDURE, J.R. et al.** *J Am Chem Soc,* 2003, vol. 125, 14716-14717
- **CONSTANTIN, T.P. et al.** *Organic letters,* 2008, vol. 10, 1561-1564
- **FILONOV, G.S.** *J Am Chem Soc,* 2014, vol. 136, 16299-16308
- **LEE, J.** *ACS chemical biology,* 2010, vol. 5, 1065-1074
- **PAIGE, J.S. et al.** *Science,* 2011, vol. 333, 642-646
- **PEI, R. et al.** *Nucleic acids research,* 2009, vol. 37, e59
- **SANDO, S.** *Chemical communications,* 2008, 3858-3860
- **SPARANO, B.A. ; KOIDE, K.** *J Am Chem Soc,* 2005, vol. 127, 14954-14955
- **DOLGOSHEINA, E.V. et al.** *ACS chemical biology,* 2014
- **STRACK, R.L. ; JAFFREY, S.R.** *Methods in enzymology,* 2015, vol. 550, 129-146
- **SUNBUL, M. ; JASCHKE, A.** *Angewandte Chemie,* 2013, vol. 52, 13401-13404
- **SATO, S. et al.** *Angewandte Chemie,* 2015, vol. 54, 1855-1858
- **PAIGE, J.S. et al.** *Science,* 2012, vol. 335, 1194
- **YOU, M. et al.** *Proc Natl Acad Sci USA,* 2015, vol. 112, E2756-2765
- **HOFER, K. et al.** *J Am Chem Soc,* 2013, vol. 135, 13692-13694
- **CHUDAKOV et al.** *Physiological reviews,* 2010, vol. 90, 1103-1163
- **YOU, M. ; JAFFREY, S.R.** *Annual review of biophysics,* 2015, vol. 44, 187-206
- **STRACK, R.L. et al.** *Nature methods,* 2013, vol. 10, 1219-1224
- **HUANG, H. et al.** *Nature chemical biology,* 2014, vol. 10, 686-691
- **WARNER, K.D. et al.** *Nature structural & molecular biology,* 2014, vol. 21, 658-663
- **ELLINGTON, A.D. ; SZOSTAK, J.W.** *Nature,* 1990, vol. 346, 818-822
- **STOLTENBURG, R. et al.** *Biomolecular engineering,* 2007, vol. 24, 381-403
- **TUERK, C. ; GOLD, L.** *Science,* 1990, vol. 249, 505-510
- **KETTERER, S. et al.** *Nucleic acids research,* 2015
- **BHADRA, S. ; ELLINGTON, A.D.** *RNA,* 2014, vol. 20, 1183-1194
- **ROGERS, T.A. et al.** *ACS synthetic biology,* 2015, vol. 4, 162-166
- **SHARMA, S. et al.** *Small,* 2014, vol. 10, 4276-4280
- **RYCKELYNCK, M. et al.** *RNA,* 2015, vol. 21, 458-469
- **PONCHON, L. ; DARDEL, F.** *Nature methods,* 2007, vol. 4, 571-576
- **SHABALA, L. et al.** *Environmental microbiology,* 2009, vol. 11, 137-148
- **PEDELACQ, J.D. et al.** *Nat Biotechnol,* 2006, vol. 24, 79-88
- **FILONOV, G.S. et al.** *Chemistry & biology,* 2015, vol. 22, 649-660
- **LAZAREV, D. et al.** *RNA,* 2003, vol. 9, 688-697
- **CHAMBERLIN, M. ; RING, J.** *The Journal of biological chemistry,* 1973, vol. 248, 2245-2250
- **SAHA, S. et al.** *Nature nanotechnology,* 2015, vol. 10, 645-651
- **HAN, K.Y et al.** *J Am Chem Soc,* 2013, vol. 135, 19033-19038

SEQUENCE LISTING

<110> Centre National de la Recherche Scientifique

<120> Fluorescent aptamers and their applications

<130> BR77979/RIM

<160> 26

<170> PatentIn version 3.5

<210> 1
<211> 14
<212> RNA
<213> Artificial Sequence

<220>
<223> first strand of the aptamer

<400> 1
ugagggucgg gucc 14

<210> 2
<211> 19
<212> RNA
<213> Artificial Sequence

<220>
<223> second strand of the aptamer

<400> 2
guugaguaga gugugggcu 19

<210> 3
<211> 38
<212> RNA
<213> Artificial Sequence

<220>
<223> example of an aptamer

<220>
<221> misc_feature
<222> (1)..(1)
<223> n varies from 4 to 100
n is a, c, g, or t

<220>
<221> misc_feature
<222> (16)..(16)
<223> n varies from 5 to 50
n is a, c, g, or t

<220>
<221> misc_feature
<222> (17)..(17)
<223> n varies from 4 to 200
n is a, c, g, or t

```
<220>
<221>  misc_feature
<222>  (17)..(17)
<223>  n varies from 5 to 50
n is a, c, g, or t

<220>
<221>  misc_feature
<222>  (18)..(18)
<223>  n varies from 5 to 50
n is a, c, g, or t

<220>
<221>  misc_feature
<222>  (38)..(38)
<223>  n varies from 4 to 100
n is a, c, g, or t

<400>  3
nugagggucg gguccnnngu ugaguagagu gugggcun                                38


<210>  4
<211>  38
<212>  RNA
<213>  Artificial Sequence

<220>
<223>  example of an aptamer


<220>
<221>  misc_feature
<222>  (1)..(1)
<223>  n varies from 4 to 10
n is a, c, g, or t

<220>
<221>  misc_feature
<222>  (21)..(21)
<223>  n varies from 5 to 50
n is a, c, g, or t

<220>
<221>  misc_feature
<222>  (22)..(22)
<223>  n varies from 4 to 200
n is a, c, g, or t

<220>
<221>  misc_feature
<222>  (23)..(23)
<223>  n is a, c, g, or u

<220>
<221>  misc_feature
<222>  (24)..(24)
<223>  n varies from 5 to 50
n is a, c, g, or t

<220>
<221>  misc_feature
<222>  (38)..(38)
```

<223> n varies from 4 to 10
n is a, c, g, or t

<220>
<221> misc_feature
<222> (39)..(39)
<223> n varies from 7 to 10
n is a, c, g, or t

<400> 4
nguugaguag agugugggcu nnnugagggu cggguccn 38

<210> 5
<211> 17
<212> RNA
<213> Artificial Sequence

<220>
<223> example of an aptamer

<220>
<221> misc_feature
<222> (1)..(1)
<223> n varies from 4 to 100
n is a, c, g, or t

<220>
<221> misc_feature
<222> (16)..(16)
<223> n varies from 5 to 50
n is a, c, g, or t

<220>
<221> misc_feature
<222> (17)..(17)
<223> n varies from 4 to 200
n is a, c, g, or t

<400> 5
nugagggucg gguccnn 17

<210> 6
<211> 22
<212> RNA
<213> Artificial Sequence

<220>
<223> example of an aptamer

<220>
<221> misc_feature
<222> (1)..(1)
<223> n varies from 4 to 100
n is a, c, g, or t

<220>
<221> misc_feature
<222> (21)..(21)
<223> n varies from 5 to 50

n is a, c, g, or t

<220>
<221> misc_feature
<222> (22)..(22)
<223> n varies from 4 to 200
n is a, c, g, or t

<400> 6
nguugaguag agugugggcu nn 22

<210> 7
<211> 69
<212> RNA
<213> Artificial Sequence

<220>
<223> example of aptamer

<220>
<221> misc_feature
<222> (1)..(10)
<223> n is a, c, g, or u

<220>
<221> misc_feature
<222> (25)..(40)
<223> n is a, c, g, or u

<220>
<221> misc_feature
<222> (60)..(69)
<223> n is a, c, g, or u

<400> 7
nnnnnnnnnn ugagggucgg guccnnnnnn nnnnnnnnnn guugaguaga gugugggcun 60

nnnnnnnnn 69

<210> 8
<211> 71
<212> RNA
<213> Artificial Sequence

<220>
<223> example of aptamer

<220>
<221> misc_feature
<222> (1)..(8)
<223> n is a, c, g, or u

<220>
<221> misc_feature
<222> (28)..(49)
<223> n is a, c, g, or u

<220>
<221> misc_feature

<222> (64)..(71)
<223> n is a, c, g, or u

<400> 8
nnnnnnnngu ugaguagagu gugggcunnn nnnnnnnnnn nnnnnnnnnu gagggucggg 60

uccnnnnnnn n 71

<210> 9
<211> 32
<212> RNA
<213> Artificial Sequence

<220>
<223> example of aptamer

<220>
<221> misc_feature
<222> (1)..(10)
<223> n is a, c, g, or u

<220>
<221> misc_feature
<222> (25)..(32)
<223> n is a, c, g, or u

<400> 9
nnnnnnnnnn ugagggucgg guccnnnnnn nn 32

<210> 10
<211> 37
<212> RNA
<213> Artificial Sequence

<220>
<223> example of aptamer

<220>
<221> misc_feature
<222> (1)..(10)
<223> n is a, c, g, or u

<220>
<221> misc_feature
<222> (30)..(37)
<223> n is a, c, g, or u

<400> 10
nnnnnnnnnn guugaguaga gugugggcun nnnnnnn 37

<210> 11
<211> 69
<212> RNA
<213> Artificial Sequence

<220>
<223> iSpinach

<400>  11
gcgacuacgg ugagggucgg guccaguagc uucggcuacu guugaguaga gugugggcuc        60

cguagucgc                                                                69

<210>  12
<211>  14
<212>  DNA
<213>  Artificial Sequence

<220>
<223>  DNA coding RNA aptamer

<400>  12
tgagggtcgg gtcc                                                          14

<210>  13
<211>  19
<212>  DNA
<213>  Artificial Sequence

<220>
<223>  DNA coding RNA aptamer

<400>  13
gttgagtaga gtgtgggct                                                     19

<210>  14
<211>  38
<212>  DNA
<213>  Artificial Sequence

<220>
<223>  DNA coding RNA aptamer

<220>
<221>  misc_feature
<222>  (1)..(1)
<223>  n varies from 4 to 100
n is a, c, g, or t

<220>
<221>  misc_feature
<222>  (16)..(16)
<223>  n varies from 5 to 50
n is a, c, g, or t

<220>
<221>  misc_feature
<222>  (17)..(17)
<223>  n varies from 4 to 200
n is a, c, g, or t

<220>
<221>  misc_feature
<222>  (18)..(18)
<223>  n varies from 5 to 50
n is a, c, g, or t

<220>
<221> misc_feature
<222> (38)..(38)
<223> n varies from 4 to 100
n is a, c, g, or t

<400> 14
ntgagggtcg ggtccnnngt tgagtagagt gtgggctn 38

<210> 15
<211> 38
<212> DNA
<213> Artificial Sequence

<220>
<223> DNA coding RNA aptamer

<220>
<221> misc_feature
<222> (1)..(1)
<223> n varies from 4 to 100
n is a, c, g, or t

<220>
<221> misc_feature
<222> (21)..(21)
<223> n varies from 5 to 50
n is a, c, g, or t

<220>
<221> misc_feature
<222> (22)..(22)
<223> n varies from 4 to 200
n is a, c, g, or t

<220>
<221> misc_feature
<222> (23)..(23)
<223> n varies from 5 to 50
n is a, c, g, or t

<220>
<221> misc_feature
<222> (38)..(38)
<223> n varies from 4 to 100
n is a, c, g, or t

<400> 15
ngttgagtag agtgtgggct nnntgagggt cgggtccn 38

<210> 16
<211> 17
<212> DNA
<213> Artificial Sequence

<220>
<223> DNA coding RNA aptamer

<220>

<221> misc_feature
<222> (1)..(1)
<223> n varies from 4 to 100
n is a, c, g, or t

<220>
<221> misc_feature
<222> (16)..(16)
<223> n varies from 5 to 50
n is a, c, g, or t

<220>
<221> misc_feature
<222> (17)..(17)
<223> n varies from 4 to 200
n is a, c, g, or t

<400> 16
ntgagggtcg ggtccnn 17

<210> 17
<211> 22
<212> DNA
<213> Artificial Sequence

<220>
<223> DNA coding RNA aptamer

<220>
<221> misc_feature
<222> (1)..(1)
<223> n varies from 4 to 100
n is a, c, g, or t

<220>
<221> misc_feature
<222> (21)..(21)
<223> n varies from 5 to 50
n is a, c, g, or t

<220>
<221> misc_feature
<222> (22)..(22)
<223> n varies from 4 to 200
n is a, c, g, or t

<400> 17
ngttgagtag agtgtgggct nn 22

<210> 18
<211> 69
<212> DNA
<213> Artificial Sequence

<220>
<223> DNA coding RNA aptamer

<220>
<221> misc_feature

<222> (1)..(10)
<223> n is a, c, g, or t

<220>
<221> misc_feature
<222> (25)..(40)
<223> n is a, c, g, or t

<220>
<221> misc_feature
<222> (60)..(69)
<223> n is a, c, g, or t

<400> 18
nnnnnnnnnn tgagggtcgg gtccnnnnnn nnnnnnnnnn gttgagtaga gtgtgggctn 60

nnnnnnnnn 69

<210> 19
<211> 71
<212> DNA
<213> Artificial Sequence

<220>
<223> DNA coding RNA aptamer

<220>
<221> misc_feature
<222> (1)..(8)
<223> n is a, c, g, or t

<220>
<221> misc_feature
<222> (28)..(49)
<223> n is a, c, g, or t

<220>
<221> misc_feature
<222> (64)..(71)
<223> n is a, c, g, or t

<400> 19
nnnnnnnngt tgagtagagt gtgggctnnn nnnnnnnnnn nnnnnnnnnt gagggtcggg 60

tccnnnnnnn n 71

<210> 20
<211> 32
<212> DNA
<213> Artificial Sequence

<220>
<223> DNA coding RNA aptamer

<220>
<221> misc_feature
<222> (1)..(10)
<223> n is a, c, g, or t

<220>
<221> misc_feature
<222> (25)..(32)
<223> n is a, c, g, or t

<400> 20
nnnnnnnnnn tgagggtcgg gtccnnnnnn nn 32

<210> 21
<211> 37
<212> DNA
<213> Artificial Sequence

<220>
<223> DNA coding RNA aptamer

<220>
<221> misc_feature
<222> (1)..(10)
<223> n is a, c, g, or t

<220>
<221> misc_feature
<222> (30)..(37)
<223> n is a, c, g, or t

<400> 21
nnnnnnnnnn gttgagtaga gtgtgggctn nnnnnnn 37

<210> 22
<211> 69
<212> DNA
<213> Artificial Sequence

<220>
<223> DNA coding iSpinach Aptamer

<400> 22
gcgactacgg tgagggtcgg gtccagtagc ttcggctact gttgagtaga gtgtgggctc 60

cgtagtcgc 69

<210> 23
<211> 15
<212> DNA
<213> Artificial Sequence

<220>
<223> oligonucleotide sense

<400> 23
ggaagacgta gcaag 15

<210> 24
<211> 22
<212> DNA
<213> Artificial Sequence

```
<220>
<223>  oligonucleotide antisense

<400>  24
cagagagatg acagagaaca ca                                              22


<210>  25
<211>  22
<212>  DNA
<213>  Artificial Sequence

<220>
<223>  oligonucleotide antisense

<400>  25
tgtgttctct gtcatctctc tg                                              22


<210>  26
<211>  64
<212>  DNA
<213>  Artificial Sequence

<220>
<223>  oligonucleotide sense

<400>  26
gtaaaacgac ggccagtgcc aagcttgcat gctaatacga ctcactatag gaagacgtag    60

caag                                                                  64
```